(19) Europäisches Patentamt
European Patent Office
Office européen des brevets

(11) EP 1 267 168 A1

(12) **EUROPEAN PATENT APPLICATION**
published in accordance with Art. 158(3) EPC

(43) Date of publication:
**18.12.2002 Bulletin 2002/51**

(21) Application number: **01912395.9**

(22) Date of filing: **15.03.2001**

(51) Int Cl.[7]: **G01N 33/53**, G01N 35/02

(86) International application number:
**PCT/JP01/02057**

(87) International publication number:
**WO 01/069249 (20.09.2001 Gazette 2001/38)**

(84) Designated Contracting States:
**AT BE CH CY DE DK ES FI FR GB GR IE IT LI LU MC NL PT SE TR**

(30) Priority: **17.03.2000 JP 2000077144**

(71) Applicant: **Unitec Co., Ltd.**
**Matsudo-shi, Chiba 270-0025 (JP)**

(72) Inventor: **TAJIMA, Hideji, UNITEC Co., Ltd.**
**Inagi-shi, Tokyo 206-0812 (JP)**

(74) Representative:
**Bohnenberger, Johannes, Dr. et al**
**Meissner, Bolte & Partner**
**Widenmayerstrasse 48**
**80538 München (DE)**

(54) **SUPPORTER FOR SUBSTANCE TO BE DETECTED, METHOD AND DEVICE FOR PROCESSING THE SAME, AND METHOD AND DEVICE FOR MANUFACTURING THE SAME**

(57) The present invention relates to the support of substances for detection, apparatus for processing same, method of processing same, apparatus for making same, and method of making same. The object of the present invention is to provide a reliable and high quality technology that can perform a series of processes, consistently, automatically and easily. A support of substances for detection of the present invention is constructed so as to comprise a flexible base member formed to be slender as thread, string or tape, a variety of substances for detection having predetermined chemical structure thereof and being fixed side by side along the length of the base member, and a supporting member for supporting the base member in a manner that enables expansion, wherein a fixed location of each substance for detection corresponds with the chemical structure thereof.

FIG. 1

(a)
10
11a
11
14
15
12
13

(b)
16
11a
11
14
15
17
18

EP 1 267 168 A1

## Description

Field of the Invention:

The present invention relates to a support of substances for detection, apparatus for processing same, method of processing same, apparatus for making same, and method of making same. Particularly, the present invention relates to all manner of fields which require inspection, analysis and examination of biopolymer like genes, immune system, proteins, amino acids, and sugars; including the fields of engineering, agricultural science incorporating foodstuffs, agricultural production, and fish processing, pharmaceuticals, the medical field incorporating hygiene, health, immunity, disease, and scientific field like chemistry and biology.

Particularly, the present invention relates to the support of substances for detection, apparatus for processing same, method of processing same, apparatus for making same, and method of making same which are suitable for analysis of genes, including mutation analysis, polymorphism analysis, mapping, base sequence analysis, and mechanism analysis.

Background of the Invention

Currently, the determination of gene base sequences uses DNA chip, wherein an oligonucleotide with a predetermined base sequence is attached to a substrate at a predetermined position. DNA chips require the preparation of a known oligonucleotide array, which is a flat surface comprising a semiconductor film or slide glass, on to which is spotted a minute quantity of suspensions of a plurality of different, known oligonucleotides, with the oligonucleotides fixed in an array pattern sequence.

In order to form a plurality of oligonucleotides on the restricted surface area of a DNA chip, it is necessary for a human operator to use a pipette apparatus and then dispense spot by spot a minute quantity of each oligonucleotide suspension onto the surface leaving a predetermined separation between adjacent spots, with avoiding cross-contamination. This DNA chip is used for various analyses and examinations of genes.

Currently, in order to determine, for instance, base sequence of an unknown target gene, a user dispenses a liquid suspending the target gene marked by luminous substance, on the DNA chip. At an interval of a certain reaction time, the user removes the remnant suspension by cleaning. Thereafter, the user determines the base sequence according to the locations where light emission is detected in the DNA chip.

In order to manufacture DNA chip, many sorts of oligonucleotides must densely be positioned plane-like in a small region. The more densely the oligonucleotides are positioned, the more closely they approach one another. Therefore, not only cross-contamination is apt to occur, but also an amount of oligonucleotide in each fixed location reduces much more.

Particularly, the reduction of the amount of oligonucleotide causes such problems that determination of locations where light emission is detected inclines to inclusion of errors and inaccuracy.

Furthermore, the reduction of the amount of oligonucleotide causes such problems that the encountering rate and efficiency of reaction are lowered and it takes long time for processing.

Further, due to plane-like arrangement of samples, there are problems that the more densely samples are positioned, the more hardly processes and automation are carried out.

Therefore, manufacturing the DNA chip requires many labor and long time, and there is such a problem that the cost of production of DNA chip increases. Particularly, in order to analyze, examine and determine the structure of unknown target substances including enormous amount of base sequences, analysis, examination and so on of large number of DNA chips are necessary.

Consequently, the present invention aims to resolve the problems outlined above. The first object is to provide a reliable and high quality support of substances for detection, apparatus for processing same, method of processing same, apparatus for making same and method of making same.

The second object is to provide a support of substances for detection, apparatus for processing same, method of processing same, apparatus for making same and method of making same which can perform a series of processes consistently, automatically and easily.

The third object is to provide a reliable and efficient support of substances for detection, apparatus for processing same, method of processing same, apparatus for making same and method of making same which can increase efficiency of reaction and encounter rate between substances for detection and target substances.

The fourth object is a support of substances for detection, apparatus for processing same, method of processing same, apparatus for making same and method of making same which can readily, easily, and inexpensively be made in large quantity.

Summary of the Invention

In order to resolve the above problems, the first aspect of the invention is a support of substances for detection comprises a flexible base member formed to be slender as thread, string or tape etc., a variety of substances for detection having predetermined chemical structure thereof and being fixed side by side along the length of the base member, and a supporting member for supporting the base member in a manner that enables expansion, wherein a fixed location of each substance for detection corresponds with the chemical structure thereof.

In this description, "substance for detection" refers to a substance which should be detected, for example, biopolymer like gene substances such as DNA, RNA and oligonucleotide, proteins, amino acids, and sugars, in order to either determine a structure of an unknown substance, or perform various analysis or examination of the target substances. When the substance for detection is a gene, "chemical structure" means a basic sequence.

The "base member" is made of flexible materials. The materials include for example, organic matters like polyethylene, polystyrene, polypropylene, urethane, inorganic matters like glass fibers, ceramics, metals, or materials combined between organic matters and inorganic matters like a film or tape made of organic matters spread by ceramic fine particles.

The base member includes a member, whose surface is covered with or is processed by or which is made of, materials for fixation like hydroxyl group, amino group, carboxyl group, epoxy group, biotin or avidin.

Suitable techniques for supporting the base member to the supporting member include winding on a core, flat winding to produce a flat plane without cores, routing through one more posts mounted in a plate, folding in, mounting, laminating or arranging. Alternatively, they include solid winding, lamination, arrangement or folding to form a cylinder, a prism, a circular cone, a pyramid or sphere.

Further, they include integration by neighboring sides thereof each other, or by forming space between them, or by sandwiching auxiliary members. The techniques to wind the base members may include not only winding with being aligned, but also loosely or irregularly winding without being aligned. In a short, transformation between integration and expansion can easily and surely be performed. Furthermore, when detection is carried out in an integrated state, locations of the substances for detection need be specified.

With regard to DNA chip, it is very difficult to position the substances for detection by dispensing etc. at correct locations densely and 2-dimensionally from the beginning. Because fixed locations are closely gathered together. In contrast, with the present invention, after that the substances for detection, for example, oligonucleotide is closely fixed on a base member in a one-dimensional state where the base member is expanded (the first integration), then the base members are integrated by closely winding (the second integration). Consequently, the support of substances for detection having a high integration rate can be obtained.

With the present invention, the integration process can be divided into two processes and the density in each process needs not be high. Therefore, the interval between fixed locations in the first integration process of the present invention can be wider than the ones in the case when the fixed locations are arranged plane-like from the beginning. Thus, each amount of substances for detection to be fixed can be larger than the case when the fixed locations are arranged plane-like from the beginning. It follows that efficiency of reaction, encounter rate, and accuracy of measurement can be improved.

Because the base members of the present invention is formed to be long and slender solid body. Therefore, though an area projected in a predetermined direction is equal, surface area in an expanded state, is much larger than that in an integrated state. Further, when the base members are expanded, process can be easily performed, and locations can be specified by detection more easily than the case of the integrated state.

Further, cavity sections like trenches and holes having bottom or no bottom, or, holding sections having foam materials, porous materials, fibrous materials, uneven materials or impregnating materials may be mounted in the base members.

The "cavity sections" include, for example, trenches or holes mounted in a side section or an upper section or convexities and concavities mounted in a side section or an upper section. The "holding sections" refers to the ones which can absorb or hold a liquid, and which is made of for example, paper, cloth, threads, strings, made of organic materials like polyethylene, polystyrene, polypropylene, urethane, or made of inorganic materials like glass, ceramics and metal.

The "materials having uneven surface" mean such materials that seem to be smooth at a glance, but that have many minute concavities and convexities or ciliary bodies in a whole, or in the side or upper sections. The materials having uneven surface is for example, such strings, threads or tapes made of organic materials on which minute ceramic particles are spread. Since the base members made of the materials having uneven surface have characteristics that contact between surfaces of the base member generates adhesion between them, the materials can use for combining the integrated base members.

It is needless to say that the base members may be the ones having an even surface being free from the cavity sections or holding sections.

Further, the cavity sections or holding sections may be arrayed in the direction of the length of the base member. Furthermore, the holding sections may include base members that are made of the one like foam materials, porous materials, fibrous materials, or impregnating materials.

Furthermore, in the case that the substances for detection are positioned in the holding sections or the cavity sections, they may be fixed by adjusting the viscosity thereof.

The amount of substances for detection held in those cavity sections or holding sections, can generally be larger than the amount of those held in the locations free from the cavity sections or holding sections. Consequently, when the reaction process is performed, the encounter rate between substances can be improved and efficiency of reaction and accuracy of detection can be improved.

As mentioned above, with the first aspect of the invention, long and slender base members which is in an expanded state at first, are supported to a supporting member of the support of substances for detection in such a manner that enables or prevents expansion. Consequently, according to the necessity, since the base members can arbitrarily chose the expanded state or integrated state, the process can be performed in optimum state. Consequently, the present invention can speed up processes, improve efficiency, facilitate the processes, and improve reliability.

For instance, process for positioning and fixing substances for detection, process for reaction, and process for detection can be sped up, improved in efficiency, facilitated, and improved in reliability, by executing positioning, fixation and detection of each substance for detection in each location, in an expanded state.

Particularly, in a state that the base members are expanded, even if the substances for detection are not densely positioned and fixed, high density can be obtained by integrating the base members. It follows that the process for positioning, fixation and reaction can be facilitated.

Furthermore, with the present invention, instead of directly handling the thin and slender minute base members, the supporting member for base members is mounted and handled and the base members are preserved, processed, and transferred, with the base members supported to the supporting member. Therefore, considering that the supporting member for base members is larger, more rigid, more compact, and more stable than the base member, the base members are facilitated to handle and protected.

Furthermore, the present invention utilizes the base member that has the substantially minute solid structure. Therefore, when the whole volume is equal, the surface of the minute solid structure is greatly larger than the one that utilizes only a plane. Hence amount of the substances for detection in each fixed location can increase, and efficiency of reaction and detection can be improved.

The second aspect of the present invention is a support of substances for detection according to the first aspect of the invention, wherein one or more marks are provided in the base members, to indicate a standard position.

For example, luminous substances like fluorescent substances are used as a "mark". The mark is used not only to indicate the standard position in the base members, but also to distinguish target substances from other substances. In the latter marks, for example, marking substances like luminescent substances that are combined with the target substances are included. Further, such substances for detection that are stuck in polystyrene minute particles of substantially same size lined up and fixed in the base members, that are covered with gold in only the upper hemisphere, may be included in the latter marks. In this case, when the substances for detection are contacted with the suspension incorporating target substances while a white light is irradiated into the surface, the color of the particles are changed by combination with the target substances. Therefore, the fixed locations combined with the target substances are directly indicated by the detected color.

It is preferable that both of the mark for indication of the standard position and the mark for discrimination of the target substances can be clearly distinguished by wavelength, strength, polarized rate, phase or life span of excitation ray or radiated ray, on the occasion of detection.

With the second aspect of the invention, since the absolute locations in the base members can easily and surely be specified, the substances for detection can surely and reliably be specified.

The third aspect of the present invention is a support of substances for detection according to the first aspect or the second aspect, wherein the base member is supported by the supporting member, with being enclosed in a defined area so that the base member can contact with a liquid and can be expanded from the area. It is preferable that the "area" is so small and compact to treat minute amount of liquid. Besides, as this area, reel, or drum is suitable. Further, the base members are looped over one more posts mounted in a plate. The area is necessary for orderly integrating and expanding the base members without tying knots or being tangled.

With the third aspect of the present invention, the base members are confined in a defined area so that they can contact with a fluid, and are supported so that they can expand from the area. Hence, reaction in a liquid suspending minute amount of a target substance can be carried out uniformly and evenly. As circumstances demand, change between the expanded state and the integrated state can easily be carried out . Further, as the base members are confined in a defined area, preservation, process and transfer etc. can easily be performed.

The forth aspect of the present invention is a support of substances for detection, according to any one of the first to third aspects of the invention, wherein the supporting member comprises a reel, wherein the reel comprises a core the base members are wound on, and two guide frames mounted on opposite ends of the core, facing one another and being permeable.

Besides, on said "core", the base member is wound in proper alignment so as to have many layer and one line, or one layer and one or more lines, or, may be wound without proper alignment.

With the fourth aspect of the invention, since the reel is used as the member for support, expansion and take-up can easily be performed, and the preservation, process, or transferring can easily be performed.

The fifth aspect of the invention is a support of substances for detection according to any one of the first to the fourth aspects of the invention, wherein the supporting member comprises a frame body, and a running support section mounted in the frame body for supporting the base member in a manner that enables running, wherein the base member can run along a running pathway defined by being supported to the running support section.

The sixth aspect of the invention is a support of substances for detection according to the fifth aspect of the invention, wherein the running support section comprises a drum mounted in the frame body in a manner that enables rotation and screwed in a peripheral thereof, wherein the frame body has an arm so that the base member can be inserted into vessels put outside of the support of substances for detection, and the base member is wound along a bottom of thread of the drum and can run through the neighborhood of a lower end of the arm by rotating the drum.

With the fifth or the sixth invention, since the base members are supported by the running support section, the substances for detection fixed in the base members can easily be transferred by running along the running pathway. Therefore, the automation can easily be realized.

Furthermore, support of substances for detection as well as the equipment relating to it can be provided at low cost and in large quantities by standardization or normalization of the frame body. Since the base members can be processed variously or transferred with the support in such a manner being supported to the running support section, the base members need not directly be touched and are protected.

Further, with these inventions, since the minute base members can be running, a process for transferring can be carried out more easily than the case that the support of substances for detection is transferred on the whole.

The seventh aspect of the invention is a support of substances for detection according to the fifth invention, wherein the running support section comprises a supply reel and a take-up reel having a core that the base member can be wound thereon, and two guide frames being permeable and mounted on the opposite ends of the core, wherein two reels are mounted in the frame body in a manner that enables rotation, the frame body has an arm that can be inserted into a vessel outside of the support of substances, and the base member is routed between two reels, so that the base member can run through the lower end of the arm.

With the seventh invention, various process can easily be carried out by running the base members between the reels, if only the lower end of the arm is inserted into a vessel etc.

The eight aspect of the invention is a support of substances for detection, according to the seventh aspect, wherein the frame body comprises a casing, an arm outwardly extending from the casing, wherein the take-up reel is mounted in the casing in a manner that enables rotation, and the supply reel is mounted in the lower end section of the arm in a manner that enables rotation. With the present invention, roller etc. is not necessary in the lower end section of the arm.

With the eighth aspect of the invention, since the time of running the base members per se for reaction can be abbreviated by mounting the drawing up reel in the lower end section of the arm, the time of process for reaction can be shortened.

The ninth aspect of the invention is a support of substances for detection according to any one of the fifth to the eighth aspects, wherein the running support section comprises one or more rollers capable of rotating and mounted in the frame body on the running pathway.

With the ninth aspect of the invention, since various running pathways can be formed with mounting rollers, various processes can be carried out. Furthermore, dry of the base members can simultaneously be carried out by absorption of a liquid with the roller.

The tenth aspect of the invention is a support of substances for detection according to the ninth aspect, comprises a protection belt sandwiched between the roller and the base member in the peripheral of the roller, that runs in a predetermined running velocity.

It is preferable that said "predetermined running velocity" are set up to be substantially equal to a running velocity of the base member, but is not limited to the case. Besides, it is preferable that "the protection belt" is formed so as to be larger than the base member in width and length.

With the present invention, the base member is not rubbed and damaged. Since the base member is not touched to the roller, cross-contamination can surely be avoided. Further, dry of the base member can be carried out by using a liquid absorbing material like paper or cloth, as the belt for protection.

The eleventh aspect of the invention is a support of substances for detection according to any one of the fifth to the tenth aspects, comprises a detection region and/or reaction region, on the running pathway of the base members, wherein the detection region is the one where substances for detection are detected, and the reaction region is the one where the reaction between the substances for detection and the target substances is carried out. In "detection", for example, an optical detection is included.

With the present invention, processes can automatically be carried out all the way through, by mounting the detection region or reaction region in the running pathway.

The twelfth aspect of the invention is a support of substances for detection according to any one of the fifth to the eleventh aspects of the invention, wherein the running pathway of the base member forms to be a loop. With this invention, reaction can easily and rapidly be performed and detection can reliably be carried out by repeatedly running the base members.

With the twelfth aspect of the invention, since the looped running pathway of the base members is used, process or measurement can repeatedly be performed only by transferring in a predetermined direction, hence it is easy to handle.

The thirteen aspect of the invention is a support of substances for detection according to any one of the fifth to the twelfth aspects, wherein the running support section comprises a coupling for combining with the outer running mechanism and running the base member. Besides, the combination with the outer running mechanism may be carried out, for example, by loading a device comprising the running mechanism with the supporting member.

With the thirteen aspect of the invention, since the coupling is mounted in the running support, the base member can easily run by combining the running support and the running mechanism, and it is easy to handle.

The fourteenth aspect of the invention is a support of substances for detection according to any one of the third to the thirteenth aspects, wherein the supporting member is made of permeable materials having a plurality of pores.

With the fourteenth aspect of the invention, since the base member can uniformly and evenly be contact with a liquid by using the supporting member made of the permeable material, process for reaction can evenly and reliably be performed.

The fifteenth aspect of the invention is a support of substances for detection according to the thirteenth aspect, wherein the supporting member comprises a spacer member for generating space around the base member, when the base member is integrated and supported.

As the spacer member of the supporting member, for example, protrusion sections inwardly extending from the guide frames of the supply reel or the spacer member of the sixteenth aspect of the invention are used so as to avoid the stick to the guide frames and generate space.

The sixteenth aspect of the invention is a support of substances for detection according to the fifteenth aspect, wherein the spacer member comprises pins, mounted in a manner that enables dismounting, so as to penetrate through holes in one guide frame, pass through a neighborhood of a peripheral of the core and reach the other guide frame, in a way to enable dismounting.

With this invention, in a state that the pins are mounted to the guide frames, the base member is wound. After winding is completed, the pins are dismounted. Hence the base member is loosely wound and space is generated around the base member.

With the fifteenth and the sixteenth aspects of the invention, the base member can uniformly and evenly be contacted with a liquid by generating space around the base members. Therefore, reliable and even process for reaction can be performed.

The seventeenth aspect of the invention is an apparatus for processing support of substances for detection, comprising plural processing regions for carrying out various processes for reaction or detection of a support of substances for detection, an installing section for installing the support of substances for detection in a manner that enables dismounting, a transfer means for transferring the substances for detection between the processing regions, with the support of substances for detection installed to the installing section, and a control section for controlling to transfer the substances for detection in a predetermined order and in a predetermined time.

Furthermore, a detection device for performing a detection in the detection region (processing region) of the running pathway, may be mounted in the apparatus for processing the support of substances for detection.

With the seventeenth aspect of the invention, the process can automatically be performed in a state that the support of substances for detection is installed in the installing section, without touched by human, all the way through.

The eighteenth aspect of the invention is the apparatus for processing the support of substances for detection according to the seventeenth aspect, the transfer means transfers the substances for detection, together with the installing section.

With the present invention, when the substances for detection are transferred together with the installing section, since the support of substances for detection is processed without being touched, more reliable process can be performed.

The nineteenth aspect of the invention is an apparatus for processing support of substances for detection according to the eighteenth aspect, wherein the installing section comprises a container for holding the support of substances for detection, wherein the container is communicating with a small diameter section capable of being inserted into the processing region like a vessel, and is mounted in the pipette device comprising a drawing/discharging mechanism capable of adjusting the pressure in the container, in a way that enables dismounting.

With the nineteenth aspect of the invention, since the process of the support of substances for detection is carried out by using the pipette device, various and diverse processes can be carried out by drawing or discharging the liquid to or from the containers holding the support of substances for detection, respectively. Further, whole processes including pre-processes can efficiently and automatically be performed by using the pipette device for dispensing reagents etc. to each vessel or for positioning a variety of substances for detection on the base members, all the way through.

The twentieth aspect of the invention is an apparatus for processing support of substances for detection according to the seventeenth aspect, wherein the installing section installs the support of substances for detection according to any one of the fifth to the sixteenth aspect of the invention in a way that the base members supported to the running support section can run, and the transfer means transfers substances for detection between the processing regions, by running only the base member in the direction of the length, in a state that the running support section is installed in the installing section.

With the twentieth aspect of the invention, only the base member of the support of substances for detection can run in the direction of the length and be transferred. Therefore, various processes can easily be carried out by only running the base member and the compact apparatus for processing can be provided as a whole.

The twenty first aspect of the invention is an apparatus for processing support of substances for detection according to the twentieth aspect, wherein the transfer means comprises a running mechanism for driving to run the base member with being connected to the running support section of the support of substances for detection.

"Connecting" is performed, for example, by connecting between the coupling mounted in the core of the reel and the rotation driving means corresponding to the running mechanism.

With the twenty-first aspect of the invention, since the base members can be run by only connecting the support of substances for detection to the running mechanism, the base member is not touched and is easy to handle.

The twenty-second aspect of the invention is an apparatus for processing support of substances for detection according to the seventeenth aspect, wherein the installing section installs the supporting member of the support of substances for detection according to any one of the fifth to sixteenth aspects of the invention, and the transfer means comprises an inter-regions transfer means for transferring the supporting member between the processing regions together with the installing section, and a running mechanism for driving to run only the base member in the direction of the length with installing the supporting member to the installing section and connecting to the running support section, wherein the substances for detection are transferred between processing regions, by using the inter-regions transfer means and the running mechanism.

With the twenty second aspect of the invention, the substances for detection are transferred by using the inter-region transferring means and the running mechanism. Therefore, when such process that includes many steps is carried out or large structure of the apparatus or large scale of space is necessary to perform, the process can rationally and efficiently be performed by separating the transference into two parts.

The twenty third aspect of the invention is an apparatus for processing support of substances for detection according to any one of the seventeenth to twenty-second aspects, wherein vessels accommodating a variety of liquids are provided in the processing regions.

The twenty fourth aspect of the invention is an apparatus for processing support of substances for detection according to the twenty-third aspect, wherein a thermostatic control means for controlling temperature in one or more vessels is installed in one or more vessels.

The twenty fifth aspect of the invention is an apparatus for processing support of substances for detection according to the twenty-fourth aspect, further comprises a vibrating means for vibrating the vessels or the support of substances for detection in the vessels.

The twenty sixth aspect of the invention is an apparatus for processing support of substances for detection according to any one of the twenty-third to twenty-fifth aspects, wherein one or more vessels accommodate a cleaning liquid.

The twenty seventh aspect of the invention is an apparatus for processing support of substances for detection according to any one of the twenty-third to the twenty-sixth aspect, comprises a dry means for drying the support of substances for detection, in one of the processing regions.

The twenty eighth aspect of the invention is an apparatus for processing support of substances for detection according to any one of the seventeenth to the twenty-seventh aspects, further comprises a detection means for detecting a change in support of substances for detection. The "change" includes light emission.

The twenty-ninth aspect of the invention is an apparatus for processing support of substances for detection according to the any one of the seventeenth to twenty-eighth aspects, further comprises an analyzer for automatically, designating relevant substances for detection on the basis of locations on the base member where a change like light emission generated by a reaction in a suspension incorporating target substances, is detected, analyzing or examining a structure of the target substances on the basis of a structure or characteristics of the substances for detection.

The "change like light emission", for example, occurs by reacting or combining any one of the substances for detection with a luminous substances for mark of the target substances.

Further, it occurs when a certain substance combined with the substances for detection changes the color or generates light emission by combining or reacting with the target substances.

With the twenty-third or the twenty-ninth aspect of the invention, diverse and efficient process can be carried out at the processing steps, by using various apparatus or performing various processes. Particularly, the process for reaction can be sped up, by vibrating the support of substances for detection per se or vessels.

The thirtieth aspect is a method of processing support of substances for detection, comprising steps of: installing a support of substances for detection to an installing section in a way that enables dismounting, transferring the substances for detection between plural processing regions for process of reaction or detection, and processing the support of substances for detection in each processing region, wherein a series of process of the substances for detection can be carried out by repeating the transferring step and the processing step.

Generally, the processing step includes detecting step for detecting a change in the substances for detection.

It is preferable that the method further comprises a dry step for drying the base members before detecting step. With the present invention, the process can automatically be carried out in a state that the support of substances for detection is installed to the installing section, without human's touch all the way through.

The thirty first aspect of the invention is a method of processing support of substances for detection according to the thirtieth aspect, wherein the substances for detection are transferred together with the installing section, at the transferring step.

With the present invention, since the base members for detection can be transferred together with the installing section, the base members are hardly touched, and the more reliable processes can be carried out.

The thirty second aspect of the invention is a method of processing support of substances for detection according to the thirtieth aspect, wherein the support of substances for detection is transferred together with an installing section, in a state that the support is installed into the installing section, at the transferring step, wherein the installing section comprises a container for holding the support of substances for detection, wherein the container is communicating with a small diameter section capable of being inserted into the processing region like a vessel, and is mounted in the pipette device comprising a drawing/discharging mechanism capable of adjusting the pressure in the container, in a way that enables dismounting.

With the thirty second aspect, since the support of substances for detection is processed by using a pipette device, various and diverse processes can be carried out by drawing or discharging a liquid to or from the containers holding the support of substances for detection, respectively. Furthermore, whole process including pre-processes, can efficiently and automatically be performed, all the way through, by using the pipette device for dispensing a reagents etc. to each vessel or for positioning each substance for detection on the base members.

The thirty third aspect of the invention is a method of processing support of substances for detection according to the thirtieth aspect, wherein the support of substances for detection according to any one of the first to the sixteenth aspects is installed so that the base member supported to a running support section of the support of substances for detection can run at the installing step, and the substances for detection are transferred between processing regions by running only the base members in the direction of the length thereof with the support of substances for detection being installed to the installing section, at the transferring step.

With that invention, the base members may be run while expanding the integrated base members integrated in the form of integration.

With the thirty third aspect of the invention, only the base member of the support of substances for detection allows to run and transfer in a direction of the length of the base member. Therefore, various processes can be carried out by running and transferring the base member, and various processes can easily be carried out and only compact apparatus for processing is necessary as a whole.

The thirty fourth aspect of the invention is a method of processing support of substances for detection according to the thirtieth aspect, wherein the base member of the support of substances for detection according to any one of the fifth to sixteenth aspects, is run in a direction of the length of the base member, and is transferred between the processing regions, by driving to run with combining a running support section to a running mechanism.

With the thirty fourth aspect of the invention, it is essential only that the support of substances for detection combines with the running mechanism in order to run the base member. Therefore the base member is not touched, and is easy to handle.

The thirty fifth aspect of the invention is a method of processing support of substances for detection according to the thirtieth aspect, wherein the supporting member of the support of substances for detection according to any one of the fifth to the sixteenth aspects, is transferred together with the installing section between the processing regions, is rotated by connecting with the running mechanism and the only base member is run and transferred in a direction of the length, so that the substances for detection are transferred between the processing regions, at the transferring step.

With the thirty fifth aspect of the invention, the base member is transferred by the inter-region transferring means and the running mechanism. Therefore, in the case that such process that have many steps are carried out or the process needs large scale apparatus or space, the processes can rationally and efficiently be carried out by separating the transferring step to two parts.

The thirty sixth aspect of the invention is a method of processing support of substances for detection according to any one of the thirtieth to the thirty-fifth aspects, the processes in the processing regions are carried out in each vessel accommodating a variety of liquids.

The thirty seventh aspect of the invention is a method of processing support of substances for detection according to any one of the thirtieth to the thirty-sixth aspects, wherein the processing step comprises a temperature control step for controlling temperature in vessels.

The thirty eight aspect of the invention is a method of processing support of substances for detection according to any one of the thirtieth to the thirty-seventh aspects, wherein the processing step comprises a vibrating step for vibrating the vessel or the support of substances for detection.

The thirty ninth aspect of the invention is a method of processing support of substances for detection according to any one of the thirtieth to the thirty eighth aspects, wherein the processing step comprises a drying step for drying the support of substances for detection to improve accuracy of detecting light emission.

The fortieth aspect of the invention is a method of processing support of substances for detection according to any one of the thirtieth to the thirty-ninth aspects, wherein the processing step comprises a reacting step for reacting a suspension incorporating target substances with the substances for detection, and a detecting step for detecting a change like light emission in the support of substances for detection, further comprises an analyzing step for designating the relevant substances for detection on the basis of the detected location of the substances for detection on the base member and analyzing a structure of a target substance on the basis of the structure of the substances for detection,

The forty first aspect of the invention is a method of processing support of substances for detection according to the fortieth aspect, wherein the detection step is carried out in a state that the base member is supported to the supporting member, in a state that the base member is expanded, or in a state that the base member is running.

With any one of the thirty sixth to the forty-first aspects, disperse and efficient processes can be carried out by using various apparatuses or carrying out various steps. Particularly, reacting step can be quickened by vibrating the support of substances for detection per se or vessels.

The forty second aspect of the invention is an apparatus for making support of substances for detection comprises a pipette device having one or more conduits and drawing/discharging means for adjusting a pressure in the conduits, a regeneration section for cleaning or exchanging the conduits, a vessel having plural liquid containing portions accommodating suspensions incorporating a variety of substances for detection and capable of being inserted by the conduit, a stage for arranging and stretching one or more base members according to any one of the first to sixteenth aspects to be dispensed, spotted, painted, or imprinted, in parallel, a displacement device which enables the movement of said conduits relative to the regeneration section, the vessels, the stage and the base members, and a control section for controlling the displacement device and the drawing/discharging means, wherein the control section controls in a manner that positions each suspension, on one or more base members, in substantially vertical direction to the length of the base members, along thin parallel lines, keeping a line from contact with neighboring lines, by repeating to draw, discharge, and displace each suspension incorporating the substances for detection, and clean or exchange the conduits.

The reel wound by the base member can be made as follows:

At first, dispensation, spotting, painting, or imprinting of the suspension is carried out on a film, with parallel lines, by the apparatus. Thereafter, the film is rolled around a core in a direction vertical to the lines and integrated. Then, the rolled film is sliced in a direction vertical to the line. Otherwise, the stretched base members are arranged on the stage in parallel. Said film is touched to the base members in a state that the parallel lines of the film are substantially vertical to the base members so that the suspensions are copied to the base members.

The forty third aspect of the invention is a an apparatus for making support of substances for detection comprises a printing device having one or more conduits, one or more reservoirs accommodating suspensions incorporating various substances for detection and communicating with the conduits, and discharging means for discharging the suspension by adjusting a pressure within the conduits or reservoirs, a regeneration section for cleaning or exchanging the conduits and reservoirs, a stage for arranging and stretching one or more base members according to any one of the first to sixteenth aspects to be printed by the printing device, a displacement device which enables the movement of said conduits relative to the regeneration section, the vessels, the stage and the base members, and a control section for controlling the displacement device and the discharging means, wherein the control section controls in a manner that positions each suspension on one or more base members, in substantially vertical direction to the length of the base members, along thin parallel lines, keeping each line from contact with the other lines, by repeating to discharge, and displace the suspensions incorporating substances for detection and clean or exchange the conduits.

The reel wound by the base member can be made as follows:

At first, printing of the suspension is carried out on a film, with parallel lines, by the apparatus. Thereafter, the film is rolled around a core in a direction vertical to the lines and integrated. Therefore, the rolled film is sliced in a direction vertical to the line. Otherwise, the base members are arranged and stretched on the stage in parallel. Said film is touched to the base members in a state that the parallel lines of the film are substantially vertical to the base members so that the suspensions are copied to the base members.

The forty fourth aspect of the invention is an apparatus for making support of substances for detection comprises one or more liquid retention tips like needles with groove, pipe, pen nib, or linear imprinting part, a regeneration section for cleaning or exchanging the liquid retention tips, a vessel having plural liquid containing portions accommodating suspensions incorporating a variety of substances for detection and capable of being inserted by the liquid retention tip, a stage for arranging and stretching one or more base members according to any one of the first to the sixteenth aspects to be painted, written, stained, or imprinted by the liquid retention tips in parallel, a displacement device which enables the movement of the liquid retention tips relative to the regeneration section, the vessels, the base member, and the stage, and a control section for controlling the displacement device, wherein the control section controls in a manner that positions each suspension on one or more base members, in substantially vertical direction to the length of the base member, along thin parallel lines, with keeping each line from contact with the other lines, by repeating to hold, and displace each suspension incorporating the substances for detection, and clean or exchange the liquid retention tips.

At first, painting, writing, staining, or imprinting of the suspension is performed on a film, with parallel lines, by the apparatus. Thereafter, the film is rolled around a core in a direction vertical to the lines and integrated. Then, the rolled film is sliced in a direction vertical to the line. Otherwise, the base member is arranged and stretched on the stage in parallel. Said film is touched to the base members in a state that the parallel lines of the film are substantially vertical to the base members so that the suspensions are copied to the base members.

The forty fifth aspect of the invention is an apparatus for making support of substances for detection according to any one of the forty-second to the forty-fourth aspects, wherein the control section the conduits or the liquid retention tips drawing, holding or storing a particular sort of suspension so as to position each suspension incorporating a substance for detection, along a parallel line with keeping each line from contact with the other lines, by repeating to dispense, paint, imprint, stain, write, or print each sort of suspension in a direction of the length of the base members, along lines, from a fixed location on the film where one or more base members are arranged or stretched in parallel and then to exchange or clean the conduits or liquid retention tips by the regeneration section, until whole sorts of suspension are completed, while shifting the predetermined location by minute length, in order.

The forty sixth aspect of the invention is a method of making support of substances for detection, comprising steps of arranging and stretching one or more base members in parallel on a plane, positioning each substance for detection on the base members by dispensing, painting, staining, inputting, writing or printing each suspension respectively incorporating a variety of substances for detection with many parallel thin lines, with keeping each line from contact with the other lines, supporting the base members fixed by substances for detection to a support member, wherein the fixed location of the substance for detection corresponds with the chemical structure thereof.

With that invention, once a variety of substances for detection are fixed to the base members, such a process as slicing step is not necessary, as is different from the case of film.

The forty seventh aspect of the invention is a method of making support of substances for detection according to the forty-sixth aspect, wherein the positioning step comprises steps of: drawing or holding a suspension incorporating a particular sort of substances for detection by displacing and inserting a conduits or a liquid retention tips to a vessel accommodating the suspensions, dispensing, painting, staining, imputing, or writing the suspensions while displacing the conduits or the liquid retention tips from a predetermined location of the base members in a direction substantially vertical to the length of the base members, and cleaning or exchanging the tube-like passengers or liquid retention tips, so that each suspension incorporating substance for detection, is positioned in parallel lines with keeping each line from contacting the other lines by repeating the above steps.

The forty eighth aspect of the invention is a method of making support of substances for detection according to the forty-sixth aspect, wherein the positioning step comprises steps of: printing a suspension incorporating a particular sort of substances for detection by displacing a conduits communicating with a reservoir accommodating the suspension, while displacing the conduits from a predetermined location of the base members in a direction substantially vertical to the length of the base members, and cleaning or exchanging the tube-like passengers and reservoirs, so that each suspension incorporating substance for detection, is positioned in parallel lines with keeping each line from contacting with the other lines by repeating the above steps.

With the forty second to the forty eighth aspect, in a state that plurality of thin expanded base members are arranged in parallel, each suspension incorporating substances for detection can surely be positioned with many parallel lines without generating cross-contamination without being intervened by human beings. Therefore, reliable support of substances for detection can be provided in large quality, at low cost.

Furthermore, with these aspects, lines of suspension incorporating substances for detection having appropriate width and concentration can easily be positioned by controlling a pressure in the conduits or displacement of the conduits or adjusting a form of the conduit or liquid retention tips. Therefore, the aspect of the invention has diversity.

Brief Explanation of Drawings

Fig. 1 is a plan showing a support of substances for detection of the first example and the second embodiments of the invention.
Fig. 2 is a plan showing a support of substances for detection of the third and the fourth embodiments of the invention.
Fig. 3 is a plan showing a support of substances for detection of the fifth embodiment of the invention.
Fig. 4 is a plan showing a base member of a support of substances for detection of the fifth embodiment of the invention.
Fig. 5 is a sectional plan of examples of the base members of embodiments of the invention.
Fig. 6 is a plan showing a support of substances for detection of the sixth and seventh embodiments of the invention.
Fig. 7 is a plan showing a support of substances for detection of the eighth embodiment of the invention.
Fig. 8 is a plan showing a measuring step for the support of substances for detection of the eighth embodiment of the invention.
Fig. 9 is a plan showing a support of substances for detection of the ninth embodiment of the invention.
Fig. 10 is a plan showing a method of processing support of substances for detection of the tenth embodiment of the invention.
Fig. 11 is a plan showing an apparatus for processing support of substances for detection and a method of processing support of substances for detection of the eleventh embodiment of the invention.
Fig. 12 is a plan showing an apparatus for processing support of substances for detection and a method of processing support of substances for detection of the twelfth embodiment of the invention.
Fig 13 is a plan showing an apparatus for making support of substances for detection and a method of making support of substances for detection of the thirteenth embodiment of the invention.
Fig. 14 is a plan showing a support of substances for detection of the fourteenth embodiment of the invention.
Fig. 15 is a plan showing a spacer member of the fifteenth embodiment of the invention.

Preferable Embodiments of the Invention

Some embodiments of the invention are explained, on the basis of drawings. These embodiments should not be interpreted to limit the invention without particular specifying.

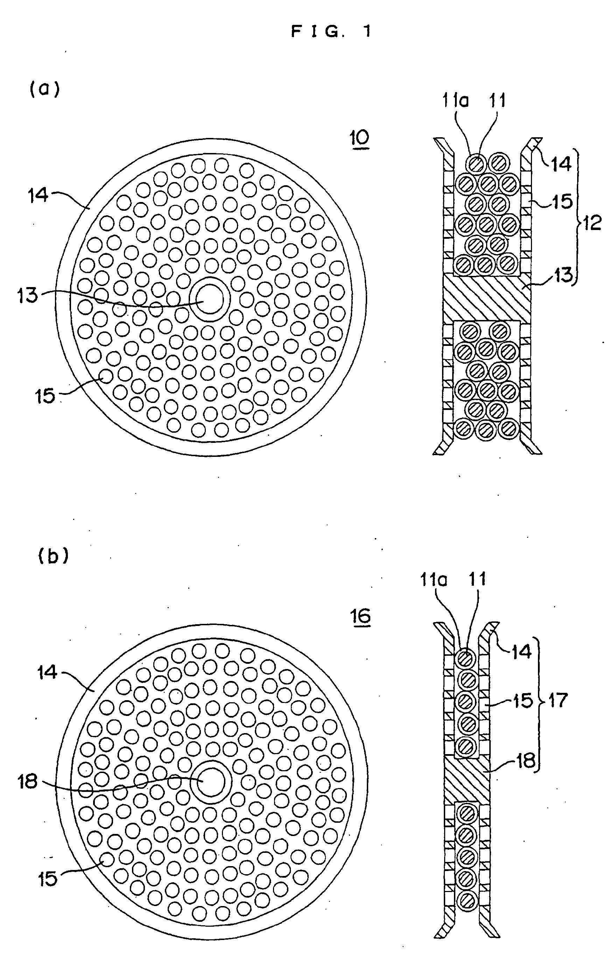

Fig. 1 (a) (b) shows examples of a support of substances for detection of the first and the second embodiments.

The support of substances for detection 10 shown in Fig. 1 (a) comprises a base member 11 that is formed to be long and slender and has pliability or flexibility, and a support member 12 that is wound on by the base member 11 in a manner that enables expansion. On the base member 11, substances for detection, for example, oligonucleotide having a predetermined base sequence, are fixed side by side along the length of the base member. Each fixed location corresponds with each base sequence thereof on the base member respectively.

Besides, the base member 11 needs be processed on the surface thereof by being coated with or be made of such materials that can fix the substances for detection. A cross section of the base member 11 is substantially circular, as circle or ellipse. Plural angular projections 11a are mounted on the outer circumstances along the circumstance at a predetermined interval.

The base member 11 is made of, for example, synthetic resin like nylon, polyethylene, or polyester. The oligonucleotide is fixed in each division partitioned by the angular projection 11a. The angular projections 11a can prevent the base member 11 from adhering to itself or adhering to the other members, and can generate space in such a manner that a liquid can flow around the base member 11.

Therefore, encounter rate between such substances for reaction like target substances suspending in a liquid and substances for detection fixed in the base member, can be improved, and reaction or combination between them can be encouraged. Furthermore, since a liquid can spread around all the substances for detection and does not contact with only a part of the substances, reliable analysis or examination can be performed.

Furthermore, the support member (12) is a reel 12 shown in Fig. 1(a). The reel 12 comprises a core 13 formed to be cylindrical, and two guide frames 14 that are formed to be disk-like and are mounted on the opposite ends of the core 13, in a state that the axes of the core 13 and the guide frames 14 coincide. In this example, the predetermined length is such width having a predetermined length (length of cylinder) that can wind the base members arranged in three lines, in maximum. Furthermore, plurality of holes 15 are mounted in the surface of the guide frames 14. A liquid can pass through the holes. It is preferable that the base member 11 is made of transparent materials so that radiation can surely be cached.

With this example, as shown in Fig. 1 (a), the base members 11 are wound and arranged in a state that the first layer has three lines, the second layer has two lines, and so on. Therefore, the base member 14 is gathered closely together, and rate of integration can be improved. Since the base member can be gathered into a compact size, process for smaller amount of liquid can be carried out.

An integrated state and a expanded state of the reel 12, are explained by offering specific examples of numerical value. The diameter of the core 13 of the reel 12 is for example, 5 mm and the diameter of the base member is for example, 0.05 mm, the length of the base member 4 is for example, 2000 mm. For example, 1000 sorts of substances for detection are fixed in a width of for example 1 mm, and at intervals of for example, 0.6 mm. For example, 30 lines of base member 11 are wound around the core 13, at intervals of for example, 0.05 mm. In these cases, the necessary width of the core 13 is, $(0.05+0.05) \times 30 = 3$ mm.

In these cases, whole area of the surface of the base member 11 is:

$$2 \pi \times 0.025 \times 2000 = \text{about } 314 \text{ mm}^2.$$

The painting area of the substances for detection is

$$2 \pi \times 0.025 \times 1 \times 1000(\text{locations}) = \text{about } 157 \text{ mm}^2.$$

The density of the positioned substances for detection is:

$$1000(\text{locations})/314 \text{ mm}^2 = \text{about } 300/100 \text{ mm}^2.$$

Furthermore, since the length of the circumference of the core 13 is:

$$2 \pi \times 2.5 = \text{about } 15.7 \text{ mm},$$

The length of the reel 12 that is wound with 30 lines is:

$$15.7 \times 30 = \text{about } 471 \text{ mm}.$$

Therefore, the base member is wound around the core 13:

$$2000 \text{ mm}/471 = \text{about } 4.24 \text{ turns}.$$

Hence, the necessary diameter of the reel 12 is:

$$5+(0.05 + 0.05) \times 4.24 = \text{about } 5.42 \text{ mm}.$$

Fig. 1 (b) shows a support of substances for detection 16 of the second embodiment. The support of substances for detection 16 is different from the support of substances for detection 10 shown in Fig. 1 (a), in that a reel 17 having a core 18 whose width (length of the cylinder) is wide enough for the base member to be wound thereon in a line, can be wound by the base member in a line. Consequently, the base member 11 can be wounded on the reel 17, without overlapping in a direction of width. For, example, when the reel 17 is made of transparent body or is formed to have a plurality of pores or openings, light emission can be detected with the base member wound on the reel 17.

In regard to the reel 17, an integrated state and expanded state are concretely explained, with offering example of numerical values.

The diameter of the core 18 of the reel 17 is for example, 3 mm, and the base member 11 is the same as the case of the one used in reel 12. Only a line of base member 11 is wound on the reel 17. In that case, necessary width of the core 18 is 0.05 mm. The necessary diameter of the reel 17 in which the core is mounted is about 11.80 mm, according to calculation of computer. If the diameter of the core 18 is about 5 mm, the necessary diameter of the reel 17 is about 12.80 mm.

In either case, the necessary diameter of the reel 17 is for example, order of about 1 mm-1 cm. The diameter of the base members is for example, order of about 0.001 mm-0.1 cm. The interval of positioned substances for detection is for example order of about 0.01 mm-1 mm. The length of the base member is for example, order of about 1 m.

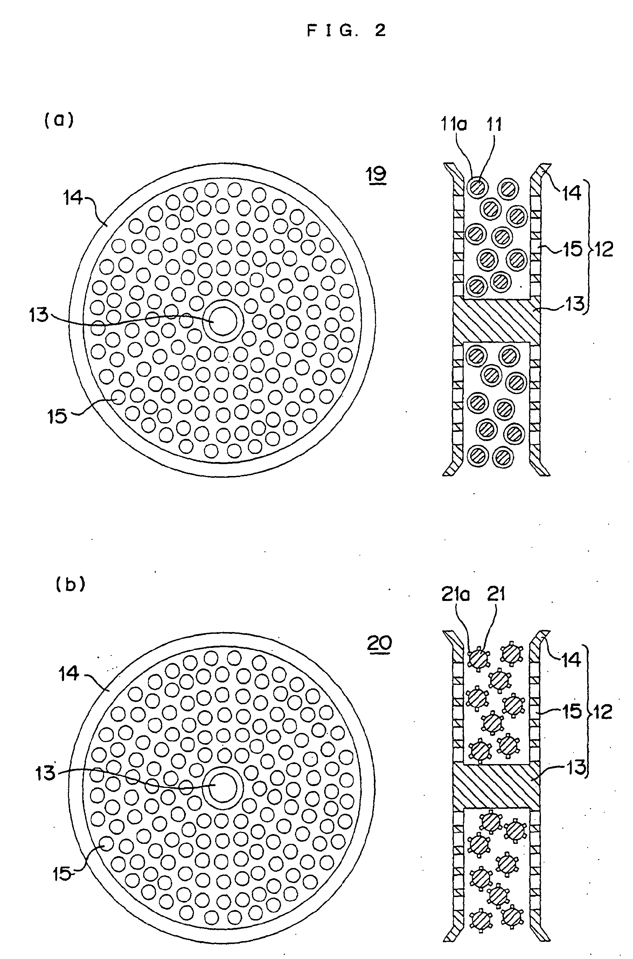

Fig. 2(a) shows a support of substances for detection 19 of the third embodiment. The support 19 of the embodiment, uses the same reel 12 shown in Fig. 1 (a). But the support 19 is different from the one shown in Fig. 1(a), in that the base member 11 is loosely wound on the reel 12 without alignment.

Hence, since sufficient space can surely be obtained around the base member, a suspension to be reacted can spread around the base member and efficiency of reaction and encounter rate of the substances for detection can uniformly improve.

Fig. 2 (b) shows a support of substances for detection 20 of the fourth embodiment. The support of substances for detection 20 of the embodiment is integrated by using the same reel 12 as the one shown in Figs. 1(a) and 2(a) and winding a base member 21 different from said base member 11 loosely, without alignment. The base member 21 is formed to be circular or substantially circular in a cross section thereof. In the outer side surface of the base member 21, projections 21a are radially projected, for instance, in six directions separated by a predetermined angle each other and arranged along the length thereof.

With this example, since sufficient space can be obtained around the base member 21 or between the base member and the guide frames 14, a liquid to be reacted can uniformly go around each substance for detection, and encounter rate between substances to be reacted can improve more.

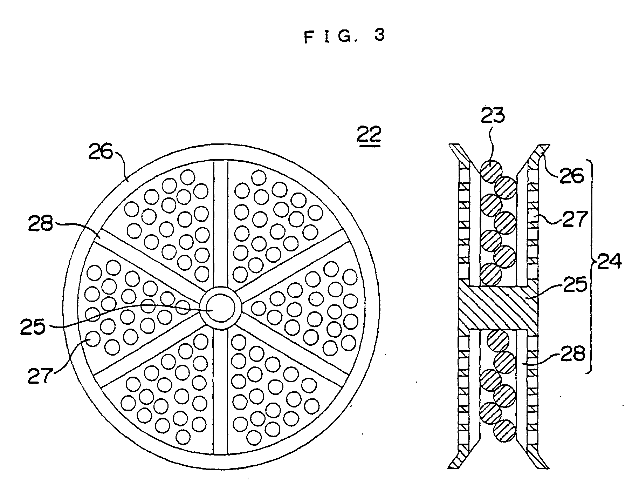

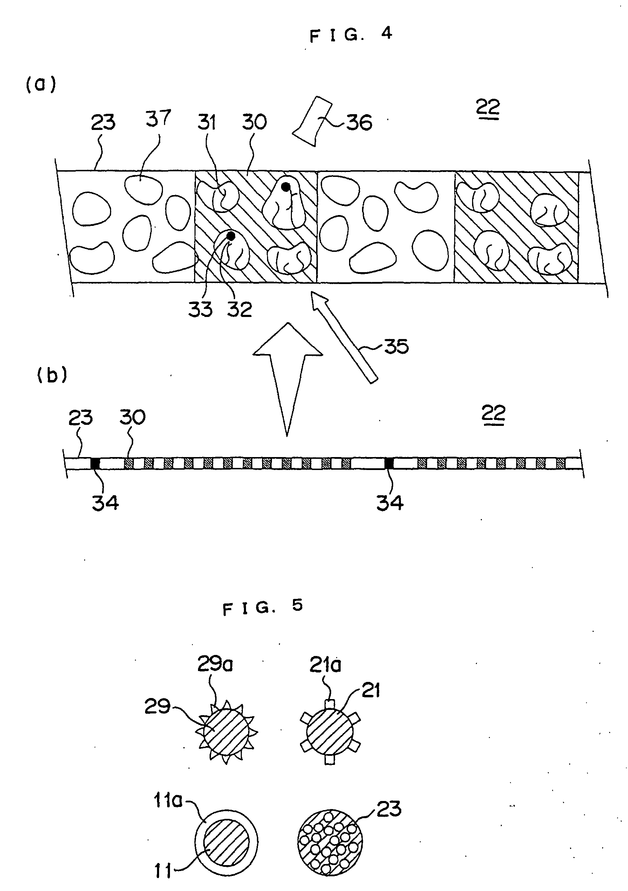

Fig. 3 shows the support of substances for detection 22 of the fifth embodiment. This support of substances for detection 22 comprises a base member 23 that is flexible and has a thin and slender shape like thread or string of circular cross section, and a reel 24 on which the base member 23 is wound in such a manner that enables or prevents expansion and which is used as a support member. As shown in Fig. 4, for example, oligonucleotides having particular base sequence, serving as substances for detection, are fixed with arranging along the length of the base member. This each fixed location and each base sequence corresponding the chemical structure, is related respectively.

As shown in Fig. 3, this support of substances for detection 22 comprises a reel 24 that constitutes the supporting member, having a core 25 formed to be cylindrical, and two guide frames 26 being permeable and mounted on the opposite ends of the core 25.

Furthermore, a plurality of holes 27 are mounted in the surface of the guide frame 26, a liquid can pass through the holes 27. The six projections 28, serving as spacer members, are projected inwardly from each guide frame 26 and are radially extended along each guide frames 26, respectively, in order to prevent the base member 23 from sticking to the guide frames 26. Those projections 28 in the opposite guide frames 23 are mounted in such a manner that are opposed face to face or alternatively.

Due to these projections 28, the base member 23 aligned with 3 lines originally is pressed inwardly so as to be away from the guide frames 26, and results in cramming into 1.5 lines. Thus the projections 28 prevent the base member from aligning with adhering to the guide frames 26, can form a space between the base member 23 and the guide frames 26, allows a liquid to flow through the space, and encounter rate between the substances for detection and a liquid to be reacted can improve.

Fig 4 (a) and 4(b) show enlarged base member 23 mounted in the support of substances for detection of the fifth embodiment, in detail. As shown in Fig. 4(a), the base member 23 is made of porous material, has a plurality of minute pores 37, and can be impregnated by a liquid. In the base member 23, certain domains 30 where particular sorts of substances for detection are fixed respectively, are mounted at predetermined intervals.

As the substances for detection 31, for example, oligonucleotide having particular sort of base sequence is used. Fig. 4 shows an example of the case when gene substances 32 like DNA fragments, used as a target substance having an unknown base sequence to be determined, being marked by a luminous substance 33 like fluorescent substances, is combined by hybridization with substances for detection 31 fixed at the domain 30.

Besides, in Fig. 4 (b), reference numeral 34 denotes a mark provided on the base member 23 at predetermined intervals, in order to indicate the standard position thereon and made of a luminous substance like fluorescent substance.

Excitation light, from for example, light source, is irradiated to the base member 23 combining with the target substance, and radiation from the luminous substance 33 and the mark 34 is received at a receiving means 36. From a location of radiation, the domain 30 on the base member 23 is specified. From the location of the domain 30 ton the base member 23, the base sequence of the oligonucleotide used as the substance for detection fixed on the domain 30 where the target substance is combined, can be determined. By determining the base sequence, the base sequence of the target substance can be determined.

Fig. 5 shows examples of various cross section of the base members 11, 23, 21, 29 in which the base members that have been explained are included. Reference numeral 29a denotes a projection mounted in the outer columnar side surface of the base member.

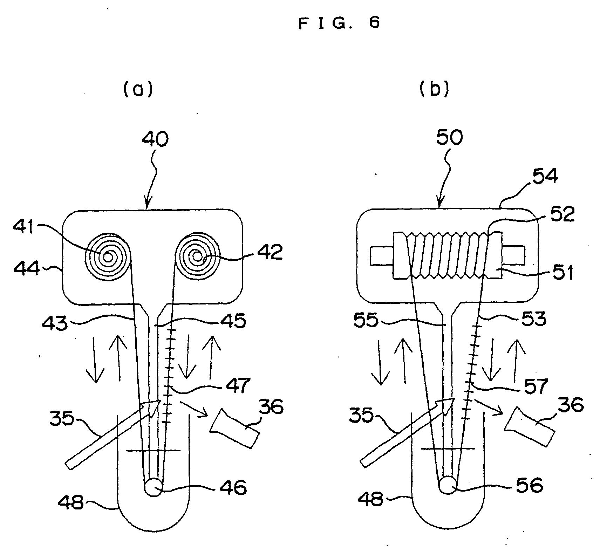

Fig. 6 (a) and (b) show support of substances for detection 40, 50 of the sixth and seventh embodiments. The support of substances for detection 40 shown in Fig. 6 (a), comprises a cassette-like support member. The support of substances for detection 40 comprises a casing used as a frame body, and an arm 45 extending outwardly from the casing 44. In the casing 44, two reels 41,42 that can be wound on by the base member 43 are fitted to the casing 44, in a manner that enables rotation. At the end of the arm 45, a roller 46 is mounted in a manner that enables rotation.

The base member 43 is wound on the reels 41, 42 in a manner that can be drawn out and taken up, and is routed through the roller 46, between the reel 41 and the reel 42. A running mechanism (not shown) is mounted to drive the reel 41 and the reel 42 to rotate alternately so that the base member 43 runs.

A plurality of sorts of substances for detection are positioned and fixed on the base member 43, along the length thereof at predetermined intervals. Inspection, examination or analysis by using the support of substances for detection 40 having the cassette like support member, can be carried out by inserting the roller 46 mounted at the end of the arm 45 into the vessel 48 accommodating a suspension incorporating a predetermined target substance. The region fixed by the substances for detection on the base member, is transferred from the one reel to the other reel by driving to rotate the reel 41 or reel 42.

The reels 41, 42 and the roller 46 correspond to the running support section for running the base member 43, and constitute the support member together with the frame body.

In order to process the support of substances for detection 40 of the embodiment, at first, this support of substances for detection 40 is installed to an installing section (not shown). The support of substances for detection 40 is transferred to the processing region where a vessel 48 accommodating a suspension incorporating target substances is set up, and the roller 46 mounted at the arm is inserted into the vessel 48.

Thereafter, the base member 43 integrated in the reel 41 is pulled by driving to rotate the reel 42, and is run along the running pathway, and whole the base members for detection can contact and react with the suspension incorporating target substance uniformly.

Next, the support of substances for detection 40 is moved to the vessel accommodating the cleaning liquid of the next processing region with being installed to the installing section. Thereafter, the base members 43 runs through the cleaning liquid, by driving to rotate the reel 41 in the direction opposite to that in the processing region.

Furthermore, in a state that the support of substances for detection 40 is installed to the installing section, the support of substances for detection is moved to the next other processing region, cleaning, drying or detecting can be carried out by driving to rotate in the direction opposite to that in the preceding processing region.

The detection is performed by irradiating the excitation light from the light source into the base member 43 while running the base member 43 along the running pathway and receiving the light emission by the receiving section, in order to detect the substance for detection combining with the target substance marked by a fluorescent substance. If each end of the base member 43 is connected with each reel, respectively, the base member 43 does not come off from the reels 41, 42.

The support of substances for detection 50 shown in Fig. 6 (b) of the seventh embodiment, comprises a cassette-like support member. The support of substances for detection 50 comprises a casing 54 used as the frame body, and an arm 55 extending outwardly from the casing 54. A drum 51 capable of rotating is fitted in the casing 54.

A roller 56 is mounted to the end of the arm 55. The base member 53 is wound along the bottom of thread screwed on the outer surface of the drum 51, and can run along the pathway routed from the drum 51, through a roller 56 to the drum 51.

Plural sorts of substances for detection are fixed side by side along the length thereof at predetermined intervals. The drum 51 and the roller 56 correspond to the running support section, and constitute the support member together with the frame body.

Since the process of the support of substances for detection 50 having a cassette-like support member is the substantially same as the case of the support of substances for detection 40 explained on the basis of Fig. 6 (a), the explanation thereof is omitted.

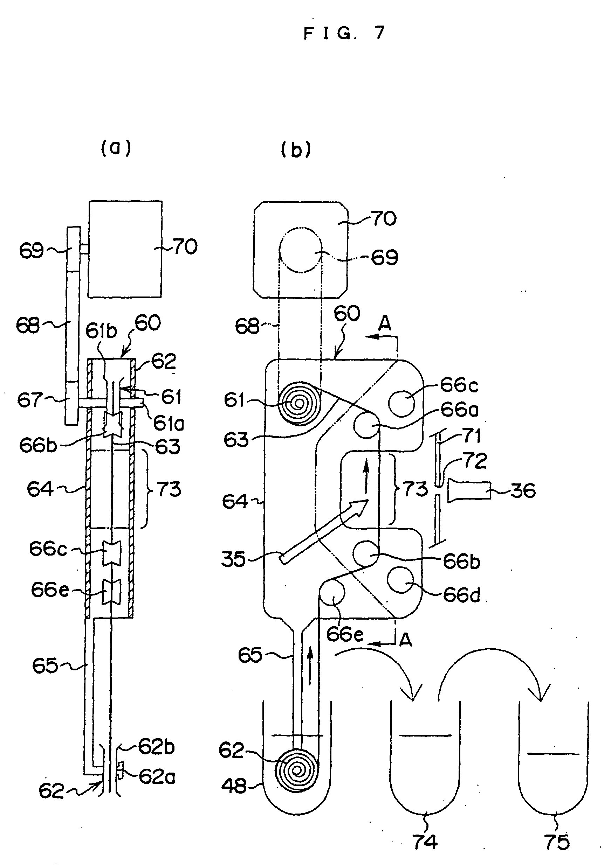

Fig. 7 shows the support of substances for detection 60 of the eighth embodiment.

This support of substances for detection 60 comprises a take-up reel 61 and supply reel 62. The base member 63 is routed through rollers 66a and 66b, between the take-up reel 61 and the supply reel 62.

Furthermore, the support of substances for detection 60 comprises a casing 64 used as the frame body and a thin arm 65 extending from the casing 64. The take-up reel 62 and rollers 66a, 66b, 66c, 66d are capable of rotating and mounted within the casing 64. The supply reel 62 capable of rotating is mounted to the end of the arm 65 in such a manner that enables insertion in the vessel 48 etc. The take-up reel 61, the supply reel 62 and the rollers 66a, 66b, 66c and 66d correspond to the running support section, and constitute a supply member, together with the frame body.

As shown in Fig. 7 (a), the take-up reel 61 and the supply reel 62 comprise cores 61a, 62a formed to be cylindrical, and two guide frames 61b, 62b being permeable and mounted on the opposite ends of the cores 61a, 62a, at established intervals, respectively.

The core 61a of the take-up reel 61 can couple to a gear 67 provided outside thereof, with the same axis. This gear 67 mechanically connects with a gear 69 of a motor 70, through a timing belt 68. The gear 67, the timing belt 68, the gear 69 and the motor 70 constitute the running mechanism.

Further, the pathway of the base member 63 between the roller 66a and the roller 66b, and outside of the casing 64, serves as a detection region 73. The substances fixed on the base member 63 are detected by obtaining a light passing through a specified position in the detection region 73. A screen 71 provided with a slit 72 is mounted in such a manner that the detection region 73 is covered except the specified position.

On the side of the screen 71 being opposite to the detection region 73, a receiving section 36 is mounted. In order to examine and analyze by using the support of substances for detection 60 having the cassette like support member, at first, the supply reel 62 wound by the base member 63 is mounted in the lower end of the arm 65, in such a manner that enables rotation. The end of the base member 63 is fixed to an empty take-up reel 61. At this stage, the substances for detection have already been fixed in the region of the base member wound on the supply reel 62.

The reaction between the target substance and the substances for detection is carried out by inserting the lower end of the arm 65 into the vessel 48 accommodating a suspension incorporating the target substance marked by a fluorescent substance.
In this case, it is preferable to vibrate the vessel or support of substances for detection 60.

After a predetermined time passes, the supply reel 62 mounted in the lower end of the arm 65, is taken out from the vessel 48, and is transferred to and inserted into the vessel 74 accommodating the first cleaning liquid. After inserting, an unnecessary suspension stuck to the supply reel 62 is removed by vibrating the vessel 74.

Thereafter, further, the supply reel 62 is taken out from the vessel 74, and is inserted into a vessel 74 accommodating the second cleaning liquid. To be sure, the vessel 75 is vibrated again, to get rid of contaminants stuck to the supply reel 62.

In a state that the supply reel 62 is soaked in the clearing liquid, in a state that the supply reel 62 is taken out from the vessel, or after drying, the base member 63 is run from the supply reel 62 to the take-up reel 61, by driving to rotate the take-up reel 61 with the motor 70.

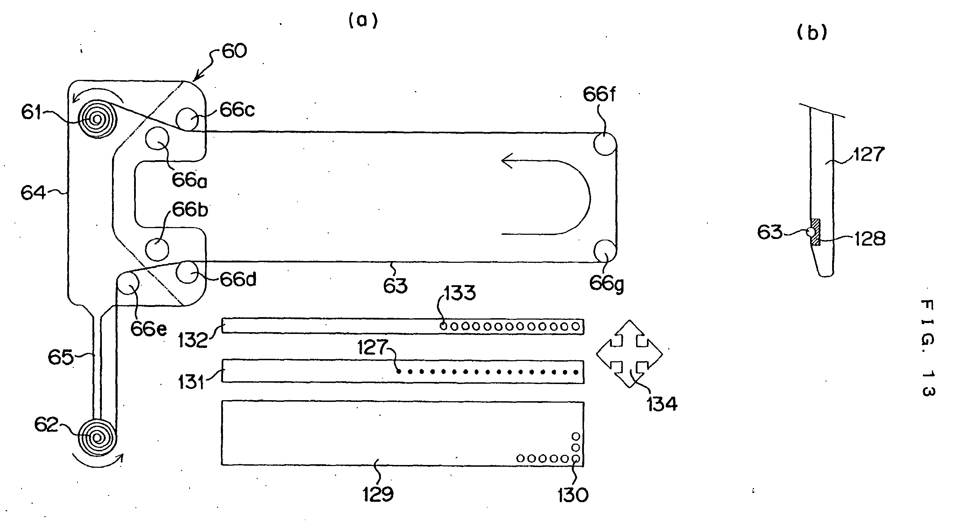

In this case, it is preferable that the roller 66b is made of such material that can absorb water when the base member passes through the roller 66b. When the base member 63 run through the detection region 73, the excitation light 35 from the radiation source is irradiated, and light emission at predetermined locations is received by the receiving means. From the result of received light emission, the structure of the target substance can be analyzed. Incidentally, the function of the rollers 66c, 66d will be explained below, on the basis of Fig. 13.

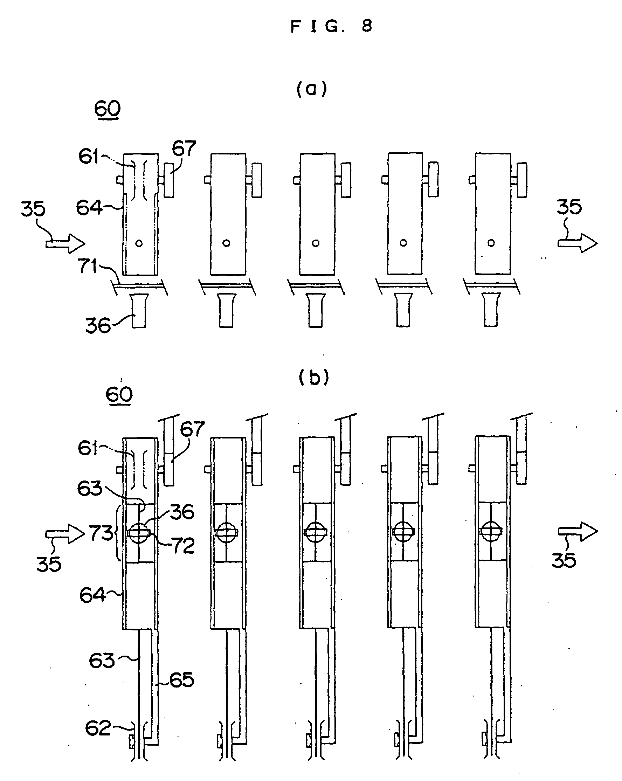

Fig. 8 shows the case when many supports of substances for detection 60 of the eighth embodiment are arranged in a row, a process of the supports of substances for detection is carried out, and then detection of light emission is carried out at the same time. In this case, a light source irradiates the excitation light 35 across the row of the casing of the supports of substances for detection into the detection regions 73, all together. Therefore, in each support of substances for detection 60, light emission can be detected or measured simultaneously, and the process and the measurement can effectively be carried out.

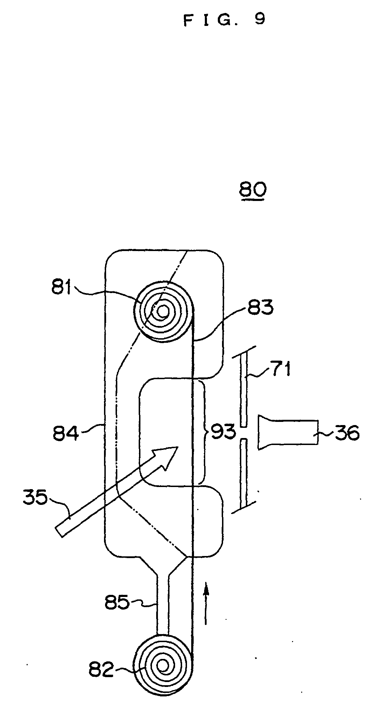

Fig. 9 shows a support of substances for detection 80 of the ninth embodiment. This support of substances for detection 80 comprises a take-up reel 81 and a supply reel 82 constituting a running support section, which can draw out and take up the base member 83 respectively. The base member 83 is routed between the take-up reel 81 and the supply reel 82, without through the rollers. Further, the support of substances for detection 80 comprises a casing 84 used as the frame body and a thin arm 85 extending from the casing 84. The take-up reel 81 is mounted in the casing 84 in such a manner that enables rotation. The supply reel 82 is mounted at the lower end of the arm 85 in such a manner that enables insertion into the vessels etc. and rotation. Besides, the reels 81, 82 correspond to the running support section, and constitute a support member together with the frame body.

Since the support of substances for detection 80 of the embodiment has such a structure that the base member 83 can run without intervening roller, the support of substances for detection 80 has a more simple structure than the one having rollers, and can surely prevent the base member 83 from being rubbed or generating cross-contaminated.

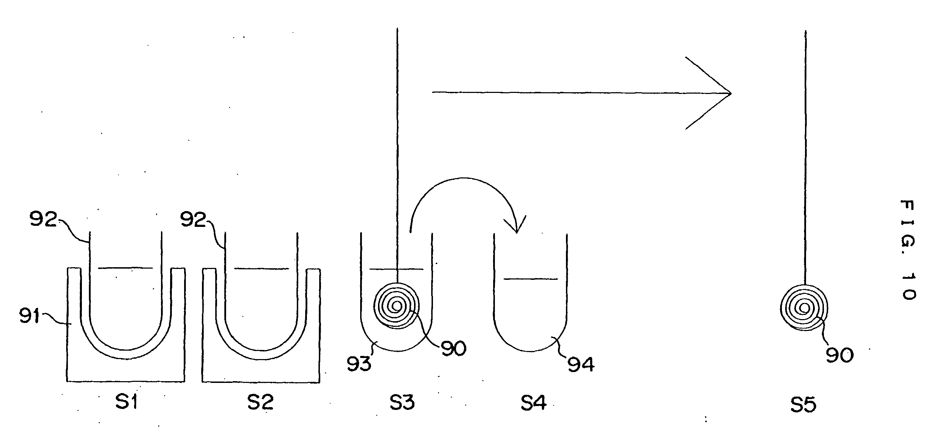

Fig. 10 shows an example of a method of processing support of substances for detection of the tenth embodiment.

The method of processing support of substances for detection is explained by giving an example of process for determining an unknown base sequence of DNA fragments used as a target substance. The method of the embodiment has the characteristics that the support of substances for detection 90 is transferred with being installed to the installing section (not shown), without being expanded.

As shown in Fig. 10, at step S1, a suspension incorporating target substances marked by fluorescent substances etc. is mixed with a predetermined reagent to obtain a prove solution. The prove solution is heated at 95° C for a few minutes, in a thermostatic tub where a Peltier effect device is mounted, beforehand. Thereafter, a direction of the current in the Peltier effect device is changed, and the prove solution is cooled at 45° C and is adjusted so as to facilitate to be hybridized.

In order to determine unknown base sequence of DNA fragments, it is needless to say that polymerization of DNA fragments, denaturation of DNA fragments and hybridization are necessary as a premise.

At step S2, integrated substance for detection 90 is transferred and inserted into a vessel 92 accommodating the prove solution. Thereafter, the vessel 92 is transferred to the thermostatic tub 91 and for instance, is kept a temperature of 65° C for about a few minutes to a few hours, or a temperature of 60° C for about a few ten minutes to a few hours in order to carry out incubation and reaction.

At step S3, after completing reaction, the support of substances for detection 90 is transferred to and inserted into a vessel 93 accommodating the first clearing liquid, and is vibrated and cleaned so that a remnant of the prove solution suspending target substances is removed, at room temperature.

At step S4, after the first cleaning, the support of substances for detection 90 is transferred to and inserted into a vessel 94 accommodating new second cleaning liquid, and is cleaned again by vibrating the vessel, and, a remnant of the prove solution is removed.

At step S5, after drying the support of substances for detection 90 by spraying dry air thereon, the light emission is detected, with the support 90 being integrated or expanded.

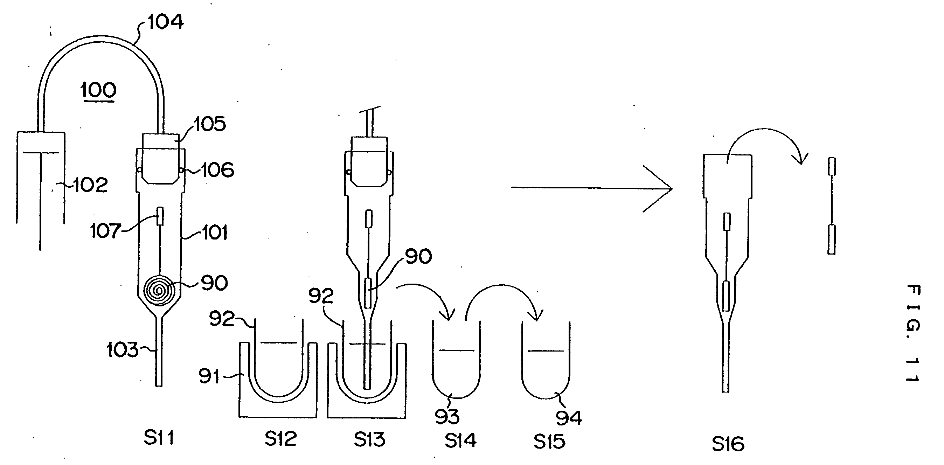

Fig. 11 shows an apparatus for processing support of substances for detection and a method of processing support of substances for detection used for determining an unknown base sequence of the DNA fragments used as the target substance.

As shown in Fig. 11, with the embodiment, a pipette device 100 is used as the apparatus for processing support of substances for detection, and the support of substances for detection 90 is installed to the installing section of the pipette device, and is transferred together with the installing section. The pipette device 100 comprises a reservoir for storing a liquid and capable of accommodating the support of substances for detection 90, used as the container, drawing/discharging mechanism 102 for adjusting a pressure within the reservoir section 101, drawing and discharging a liquid, like cylinder, and a small diameter section 103 communicating with the reservoir section 101 and capable of being inserted to a vessel set up in a processing region being out of the pipette device.

The reservoir section 101 is fitted to a nozzle 105 communicating through a pipe 104 with the drawing/discharging mechanism in such a manner that enables dismounting. A sealing member like O-ring, is mounted in the nozzle 105, to prevent from leaking a liquid.

Within the reservoir section 101, the support of substances for detection 90 is installed to an installing section 107 provided in an inner wall of the reservoir section 101.

Though not shown in drawings, this apparatus comprises, a transfer means for transferring the reservoir section 101 and the small diameter section 103, and a control section for controlling the transfer means and the drawing/discharging mechanism.

This control section comprises an information processing device having an imputing section for instructing an operation or imputing data by operator, an output section for displaying or outputting a result of operation and contents of instruction or data to the operator, a data storing device, and an arithmetic unit like CPU for analyzing the instruction of operation, instructing to each unit, displaying the result of operation and analyzing.

With the method of processing support of substances for detection of the eleventh embodiment, at step S11, the support of substances for detection 90 is installed into the reservoir section 101 of the pipette device 100. The reservoir section 101 is fitted to the nozzle 105.

At step S12, after heating a vessel 92 accommodating a prove solution mixing a suspension incorporating target substances marked by fluorescent subjects with predetermined reagents, by the thermostatic tub 91 having Peltier effect devices, at temperature of 95° C, for a few minutes, beforehand, the suspension is adjusted by reversing the direction of current in the Peltier effect devices and by cooling at a temperature of 45° C so that hybridization is apt to occur in the solution.

At step S13, the support of sections for detection 90 is transferred together with the reservoir section 101. The small diameter section 103 of the pipette device 100 is inserted into the vessel 92. The vessel 92 keeps a temperature, for example, of 65 ° C for about a few minutes to a few hours, or a temperature for example, of 60° C for about a few ten minutes to a few hours, by the thermostatic tub 91 so that the support is incubated and reacted. In this case, the pipette device 100 repeats to draw a liquid in the vessel 92 and discharge the liquid at regular intervals, so that the support of substances for detection 90 within the reservoir 101 can contact with the liquid having the temperature..

At step S14, after completing the reaction, the support of substances for detection 90 is transferred to the vessel 93 accommodating the first cleaning liquid having a room temperature, together with the reservoir section 101, and the small' diameter section 103 of the pipette device 100 is inserted into the vessel 93. Then the support 90 is cleaned with repeating to draw and discharge the cleaning liquid by the pipette device 100, so that a remnant of the prove solution suspending the target substance can be removed.

At step S15, after the first cleaning, the support 90 is transferred to the vessel 94 accommodating the new second cleaning liquid together with the reservoir section 101. The small diameter section 103 is inserted into the vessel 94. Then the support of substances for detection 90 is cleaned with repeating to draw and discharge the cleaning liquid by the pipette device 100, so that a remnant of the prove solution can be removed further.

At step S16, the support of substances for detection 90 whose cleaning is completed, is dismounted and taken out from the reservoir section 101. Then the support is dried with being blown by dry air, the dried support 90 is expanded and the optical detection is carried out.

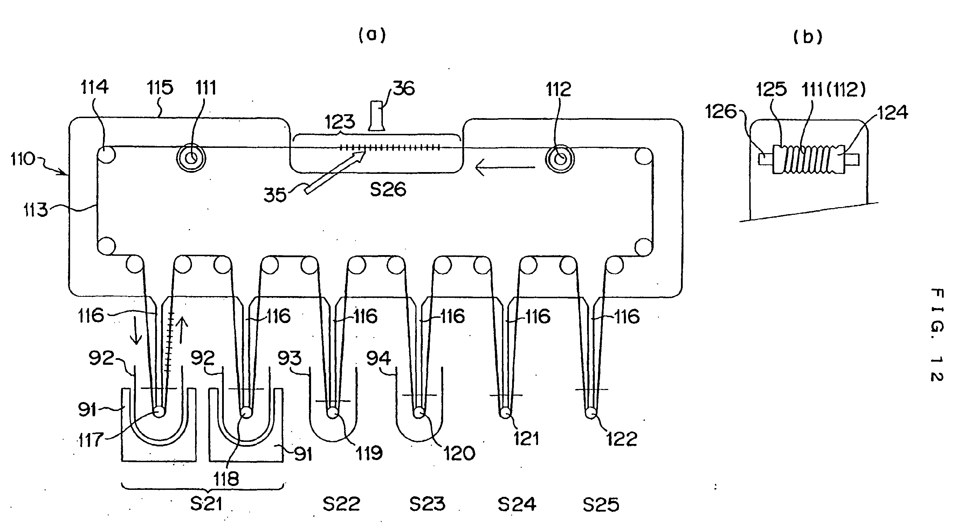

Next, the support of substances for detection 110 of the twelfth embodiment and the method of processing the support are explained oh the basis of Fig. 12.

The support of substances for detection 110 of the twelfth embodiment, comprises two drums 111, 112 capable of drawing out and taking up a base member 113. The base member 113 is formed to be thin and slender string-like, is wound on the drum 111 and drum 112, and is looped with a routed running pathway through sixteen rollers 114 and six rollers 117-122.

Further, the support of substances for detection 110 comprises a casing 115 used as the frame body, and six thin arms 116 extending downwardly from the casing 115.

The drums 111, 112 and the sixteen rollers 114 are mounted within the casing 115 in such a manner that enables rotation. Six rollers 117-122 are mounted at the lower end of the arms 116 in such a manner that enables rotation. The lower ends of the arms 116 are formed to be small enough to be able to be inserted into the vessels 92,92, 93, 94. The rollers 117-120 mounted at the lower end of the arms 116 are inserted into the vessels 92, 92, 93, 94 respectively, and the rollers 121, 122 are mounted in the air.

Further, many sorts of substances for detection are positioned in a direction of the length, at regular intervals. A part of the running pathway of the base member 113 is set up in such a manner that passes through a detection region 123 which is mounted out of the casing 115.

As shown in Fig. 12 (b), the drum 111 (or drum 112) comprises a drum-like core 124, a rotational axis 126 coincident with that of the core 124, and a bottom of thread 125 screwed in the outer surface of the core 124, and the base member 113 is wound along the bottom of the thread 125.

The drums 111, 112 and rollers 117-122 correspond to the running support section, and constitute the support member together with the frame body. Though not shown in the drawings, this apparatus comprises a running mechanism for driving to rotate the drum 111 or the drum 112 and run the base member 113 of the support of substances for detection 100, with loading the support of substances for detection 110 thereto, and a control section for controlling the running mechanism.

Next, the method of processing support of substances for detection of the twelfth embodiment using the support of substances for detection 110 is explained.

At step S21, after heating the vessel 92 accommodating the prove solution mixing a suspension incorporating the target substances marked by the fluorescent substances with a reagent, at a temperature of 95° C, for a few minutes by the thermostatic tub 91 having Peltier effect devices, the prove solution is adjusted by reversing the direction of current in the Peltier effect devices and cooling at a temperature of 45° C so that hybridization is apt to occur in the solution.

Next, the vessel 92 is kept at a temperature of, for example, 65° C, by the thermostatic tub 91. In this state, an incubation and a reaction of a portion passing through the rollers 117, 118 of the base member 113 are carried out for about a few minutes to a few hours respectively, or at a temperature of 60° C, for a few 10 minutes to a few hours, respectively. While keeping the rotational velocity of the drum 111,112, the substances for detection fixed in the base member 113, are transferred by driving to rotate the drums 111, 112.

At step S22, with the velocity, the portion of the base member 113 that the reaction is completed passes through the roller 119 which is inserted into the vessel 93 accommodating the first cleaning liquid. On that occasion, a remnant of the prove solution suspending the target substance is removed by cleaning the portion of the base member 113 and vibrating the vessel.

At step S23, with the velocity, when the portion of the base member 113 that the first cleaning is completed passes through the roller 120 which is inserted into the vessel 94 accommodating the second cleaning liquid. On that occasion, the portion of the base member 113 is cleaned by vibrating the vessel, and remnant of the prove solution suspending the target substance that cannot be cleaned out at the first cleaning is removed.

At step S24, with the velocity, when the portion of the base member 113 that the second cleaning is completed passes through the roller 121, the portion is dried with blowing dry air.

At step S25, with the velocity, the portion of the base member 113 dried at the step, is further blown by a dry air, so that water cannot be removed by the first dry, is further removed thoroughly.

At step S26, with the velocity, when the portion of the base member 113 that the dry is completed, passes through the detection region 123, the excitation light 35 is applied to the portion, and light emission is received by the receiving section 36,

With the method of the embodiment, since the support of substances for detection 110 per se needs not be moved and only the base member 113 is run by driving to rotate the drums 111,112, a structure of the transfer means can be simplified, and the apparatus can be downsized. The method of the embodiment is effective in the immunoassay.

Next, the apparatus for making support of substances for detection and the method of making support of substances for detection of the thirteen embodiment on the basis of Fig. 13.

The apparatus of the thirteenth embodiment is used for positioning the substances for detection on the base member 63 of the support of substances for detection 60 having a cassette-like support member.

As shown in Fig. 13(a), the apparatus comprises, a microplate-like vessel 129 having many wells 130 accommodating a suspension incorporating a variety of substances for detection like oligonucleotide respectively, a head 131 where plural needles with groove 127 are lined up, a cleaning tub 132 having cleaning sections 133 for cleaning the needles with groove 127, and a transfer means 134 for transferring the head 131 among the microplate-like vessel 129, the cleaning tub 132 and the place where the base members 63 are expanded and stretched, in directions of XYZ-axes.

Each cleaning section 133 of the cleaning tub 132 is structured to be able to remove used cleaned liquid and be supplied new cleaning liquid all the time, individually (a method of overflow).

Fig. 13(b) shows a state that the needle with groove 127 really positions the suspension incorporating the substances for detection to the base member 63, schematically. In the drawing, the reference numeral 128 shows a groove mounted in the needle with groove 127, where the suspension is held.

Though is not shown in drawings, the apparatus comprises a control section for controlling the transfer means 134. This control section comprises an information processing device having an input section for instructing an operation or inputting data by operator, an output section for displaying or outputting a result of operation and contents of instruction or data to the operator, a data storing device, and an arithmetic unit like CPU for analyzing the instruction of operation, instructing to each unit, displaying the result of operation, and analyzing.

In order to stick the substances for detection in the base member 63 by using the apparatus, such a running pathway of the base members 63 that routes from the reel 62 through the rollers 66e, 66d, 66c mounted within the casing 64, and through the rollers 66f, 66g, to the reel 61, serving as the running support section, needs be set up.

Next, the head 131 is transferred to the microplate-like vessel 129 by instructing the transfer means 134. Then needles with groove 127 are inserted into wells simultaneously, so that the suspensions accommodated in the well 30 are held by the grooves 128 of the needles 127. Thereafter, the head where needles with groove 127 holding the suspension are mounted by is transferred to the place where the base members 63 are stretched, and the grooves 128 of the needles 127 are brought into contact with the base member 63.

After completing the contact, the head 131 is transferred to the cleaning tub 132 by the transfer means 134, and each needle with groove 127 is inserted into each cleaning section 133 and cleaned. On that occasion, the base members 63 are run such a length that the suspension incorporating the substances for detection are stuck by the head 131, by driving to rotate the reel 61 of the cassette-like support of substances for detection.

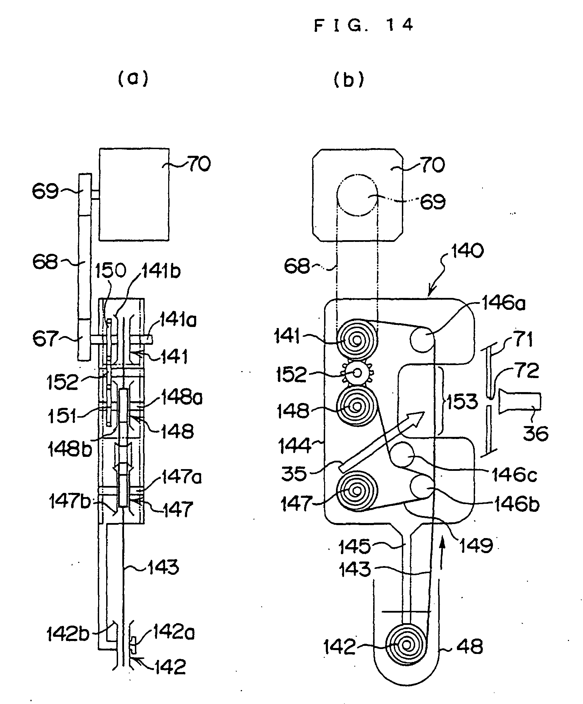

If the base members are tape-like and have the thickness of about 0.001 mm, the width of about 0.03 mm and the length of 1m, and the interval between neighboring substances for detection of about 1 mm, about 1000 substances for detection can be positioned in a projected area of 1 $mm^2$ with the base member being integrated. Therefore, in the integrated state (though the integrated state is not always necessary) the base member has a high density of substances for detection of 1000/$mm^2$. However, if the substances for detection are fixed in the surface of the base member (the thickness is about 0.03 mm, the width is about 0.001 mm, and the length is about 1 m), the surface area of the base member is about 60 $mm^2$, and the density is about 16/$mm^2$ which is low density. Therefore, in a expanded state, the positioning and fixation can be carried out in a lower density. It shows how easy to be able to position the substances in an expanded state. The support of substances for detection of the fourteenth embodiment is explained on the basis of Fig. 14. Since the same reference numeral as the one explained in Fig. 7 denotes the same one, the explanation about the one is omitted.

As shown in Fig. 14(b), this support of substances for detection 140 comprises a take-up reel 141 and a supply reel 142 capable of taking up and supplying a base member 143, respectively. The base member 143 is routed between the take-up reel 141 and the supply reel 142, through the rollers 146a, 146b. In an initial state, the greater part of the base member 143 is wound on the take-up reel 142.

This support of substances for detection 140 further comprises two protection reels 147, 148. Between the protection reel 147 and the protection reel 148, a thin and slender protection band 149 having a width and a length larger than the width and length of the base members and made of flexible absorbable material like paper or cloth is routed through the roller 146b and the roller 147. In an initial state, the greater part of the protection band 149 is wound on the protection reel 148.

Further, the support of substances for detection 140 comprises a casing 144 serving as the frame body, and a thin arm extending from the casing 144. The reels 141, 147, 148, and rollers 146a, 146b, 146c are mounted within the casing 144 in such a manner that enables rotation. The reel 142 is mounted to the lower end of the arm 145 in such a manner that enables insertion into the vessel 48 etc. and rotation.

As shown in Fig. 14(a), the reels 141, 142, 147, 148 comprise cores 141a, 142a, 147a, 148a formed to be cylindrical, and two guide frames 141b, 142b; 147b, 148b which are mounted on an opposite ends of the cores at a predetermined interval and through which a liquid can be permeate, in such a manner that enables rotation.

A coupling section for connecting with the outer gear 67 in such a manner that the axis of the gear coincides with the core 141a, is mounted in the core 141a of the take-up reel 141. A gear 150 is mounted in the core 141a of the take-up reel 141 in such a manner that the axis of the gear coincides with that of the core 141a. The gear 150 is mounted within the casing 144.

A gear 151 is mounted within the casing 144, in such a manner that an axis of the core 148a of the protection reel 148 coincides with that of the core 148a. These gear 150 and gear 151 are connected, through the gear 152 mounted within the casing 144. Besides, for instance, the tooth number of the gear 150 agrees to that of the gear 151.

The take-up reel 141, the supply reel 142, the roller 146b, the roller 146a and the gears 150, 151, 152 correspond to the running support section. The gear 67, a timing belt 68, a gear 69 and a motor 70 that are separated from the support of substances for detection 140, correspond to the running mechanism.

In consideration of the support of substances for detection of the embodiment constructed as mentioned above, at an initial state, the base member 143 ought to be wound on the take-up reel 142, and the protection belt 149 ought to be wound on the protection reel 147. In this state, when the reel 141 is driven to rotate by the motor 70, the base member 143 runs along the pathway from the reel 142 through the roller 146b and the detection region 153 to the reel 141.

In this case, a torque from the motor 70 drives to rotate the protection reel 148, through the gears 150, 152, 153, and runs the protection belt 149, along the pathway from the reel 147, through the roller 146b and the roller 146c to the reel 148, with the velocity substantially equal to that of the base member 143.

Therefore, since the protection belt 149 contacts with the base member 143, with the equal velocity, in such a manner that the belt is sandwiched between the base member 143 and the roller 146b at the outer circumference of the roller 146b, rub generated by a relative velocity between the base member 143 and the protection belt 149 can be avoided. Further, since the protection belt does not contact with the roller 146b, cross-contamination can be prevented.

Further, since the protection belt 149 can absorb the water stuck to the base member 143 and can dry the base member 143, the reliable detection can be carried out in the detection region 153 where the base member passes after being dried. The reels 141, 142 and the rollers 146a, 146b, 146c correspond to the running support section , and constitute the support member together with the frame body.

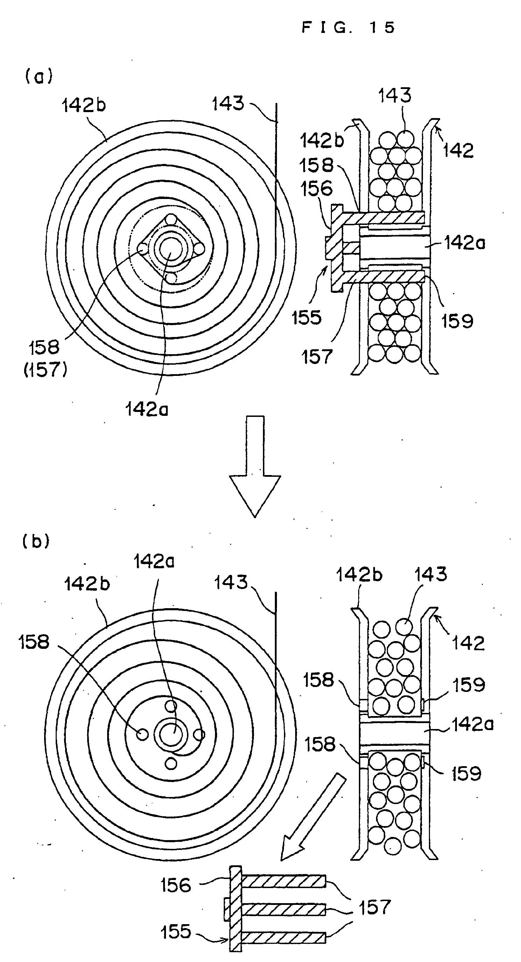

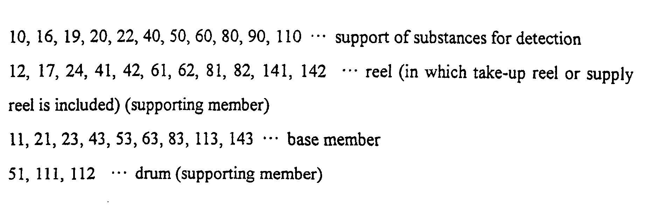

A spacer member of the fifth embodiment is explained on the basis of Fig. 15. As the same reference numerals as the reference numerals having already been explained on the basis of Fig 14 indicate the same, the explanation is omitted.

Fig. 15 (a) shows the spacer member 155 of the embodiment equipped to for instance, the supply reel 142. Fig. 15 (b) shows the spacer member 155 dismounted from the supply reel 142.

The spacer member 155 comprises a substrate 156, and four pins projecting from the substrate 156. The spacer member 155 is equipped in such a manner that pierces through four holes made in the one guide frame 142b, passes a location near by the outer circumference of the core 142a and reaches four depressions formed in the other guide frame 142b. These four holes 158, four pins 157, and four depressions are formed to have a central angle of 90° respectively.

As shown in Fig. 15 (a), in order to use the spacer member 155, at an initial state, the spacers member 155 is equipped by piercing four pins through four holes 158 made in the guide frame 142b respectively. Keeping that state, the base member 143 is wound in aligned state, while tensioning the base member 143 and tying up the core 142a and four pins 157 in a bundle.

After completing to wind, as shown in Fig. 15 (b), the spacer member 155 is dismounted from the reel 142 with the base member 143 being supported to the reel 142. Then, since tension imposed to the base member 143 is removed, the base member 143 is loosely wound on the core 142a.

With the embodiment, since sufficient space is generated around the base member 143, a suspension incorporating target substances can spread around the base member and reaction can be carried out uniformly. With the embodiment, loose winding state can be obtained more easily than the case where the base member are wound without tension from the beginning.

Although the above mentioned embodiment is specifically explained for a complete and clear disclosure of the invention, the appended claims are not to be thus limited. The appended claims can be construed as embodying all modifications and alternative constructions that may occur to one skilled in the art which fairly fall within the basic teaching herein set forth. For instance, with the above embodiments, the case when the substances for detection are oligonucleotides, and the target substance is DNA fragments is explained, the case of the other biopolymer like genetic substances, proteins, amino-acids, immune substances, or sugars is not excluded.

Constituents, substance, apparatus, components, or steps can be combined arbitrarily while changing appropriately. Further, with the above embodiment, analysis of only the target substance marked by fluorescent substances is explained (disclosed). However, the invention is not limited to the case. For instance, analysis of the target substance may be carried out by using such substances for detection that are stuck in polystyrene minute particles of substantially same size lined up and fixed in the base member, that are covered with gold in only the upper hemisphere. In this case, when the substances for detection are brought into contact with the suspension incorporating the target substances, the color of the particles are changed by a combination with the target substances, while a white light is irradiated into the surface. Therefore, analysis of target substances can be carried out on the basis of detected color.

Furthermore, though the base member whose cross section is substantially circle, is explained, the invention is not limited to the case. For instance, a tape-like base member is not excluded.

With the above mentioned embodiments, though only the case where the substances for detection are positioned by attaching the liquid holding pen nib to the base member is disclosed, the invention is not limited to the case. For instance, the substances for detection can be positioned by using a pipette device or a printing device, naturally.

Furthermore, with the above embodiments, the case where the detection is carried out by irradiating the excitation light from the light source. The invention is not limited to the case. For instance, the case where irradiation of the excitation light is not necessarily is not excluded.

Besides, in order to make the reel wound by base member, for instance, each suspension incorporating each sort of substance for detection is dispensed, painted, imprinted, written or printed on a film, without contacting each other, in many parallel lines having thin width, by using the apparatus for making support of substances for detection. The film where the substances are fixed, is rolled on a cylindrical core in the direction vertical to the lines to integrate the substances. The rolled film is sliced together with the core, in a direction vertical to the axis of the core. Two guide frames are fitted to the one obtained by slicing the rolled film, on the opposite ends thereof and the reel wound by the base member is obtained. The cassette-like or cartridge-like supports of substances for detection can easily be obtained by mounting the reel to the frame body, in large quantities and in low cost.

## Claims

1. A support of substances for detection comprising a flexible base member formed to be slender as thread, string or tape etc., a variety of substances for detection having predetermined chemical structure thereof and being fixed side by side along the length of the base member, and a supporting member for supporting the base member in a manner that enables expansion, wherein a fixed location of each substance for detection corresponds with the chemical structure thereof.

2. A support of substances for detection according to claim 1, wherein one or more marks are provided in the base member, to indicate a standard position.

3. A support of substances for detection according to claim 1 or claim 2, wherein the base member is supported by the supporting member, with being enclosed in a defined area so that the base member can contact with a liquid and can be expanded from the area.

4. A support of substances for detection according to any one of claim 1 to claim 3, wherein the supporting member comprises a reel, wherein the reel comprises a core the base members are wound on, and two guide frames mounted on opposite ends of the core, facing one another and being permeable.

5. A support of substances for detection according to any one of claim 1 to claim 4, wherein the supporting member comprises a frame body, and a running support section mounted in the frame body for supporting the base member in a manner that enables running, wherein the base member can run along a running pathway defined by being supported to the running support section.

6. A support of substances for detection according to claim 5, wherein the running support section comprises a drum mounted in the frame body in a manner that enables rotation and screwed in a peripheral thereof, wherein the frame body has an arm so that the base member can be inserted into vessels put outside of the support of substances for detection, and the base member is wound along a bottom of thread of the drum and can run through the neighborhood of a lower end of the arm by rotating the drum.

7. A support of substances for detection according to claim 5, wherein the running support section comprises a supply reel and a take-up reel having a core that the base member can be wound thereon, and two guide frames being permeable and mounted on the opposite ends of the core, wherein two reels are mounted in the frame body in a manner that enables rotation, the frame body has an arm that can be inserted into a vessel outside of the support of substances, and the base member is routed between two reels, so that the base member can run through the lower end of the arm.

8. A support of substances for detection, according to claim 7, wherein the frame body comprises a casing, an arm outwardly extending from the casing, wherein the take-up reel is mounted in the casing in a manner that enables rotation, and the supply reel is mounted in the lower end section of the arm in a manner that enables rotation.

9. A support of substances for detection according to any one of claim 5 to claim 8, wherein the running support section comprises one or more rollers capable of rotating and mounted in the frame body on the running pathway.

10. A support of substances for detection according to claim 9, comprises a protection belt sandwiched between the roller and the base member in the peripheral of the roller, that runs in a predetermined running velocity.

11. A support of substances for detection according to any one of claim 5 to claim 10, comprises a detection region and/or reaction region, on the running pathway of the base member, wherein the detection region is the one where substances for detection are detected, and the reaction region is the one where the reaction between the substances for detection and the target substances is carried out.

12. A support of substances for detection according to any one of claim 5 to claim 11, wherein the running pathway of the base member forms to be a loop.

13. A support of substances for detection according to any one of claim 5 to claim 12, wherein the running support section comprises a coupling for combining with the outer running mechanism and running the base member.

14. A support of substances for detection according to any one of claim 3 to claim 13, wherein the supporting member is made of permeable materials having a plurality of pores.

15. A support of substances for detection according to any one of claim 3 to claim 14, wherein the supporting member comprises a spacer member for generating space around the base member, when the base member is integrated and supported.

**16.** A support of substances for detection according to claim 15, wherein the spacer member comprises pins, mounted in a manner that enables dismounting, so as to penetrate through holes in one guide frame of the reel, pass through a neighborhood of a peripheral of the core and reach the other guide frame.

**17.** An apparatus for processing support of substances for detection, comprising plural processing regions for carrying out various processes for reaction or detection of a support of substances for detection, an installing section for installing the support of substances for detection in a manner that enables dismounting, a transfer means for transferring the substances for detection between the processing regions, with the support of substances for detection installed to the installing section, and a control section for controlling to transfer the substances for detection in a predetermined order and in a predetermined time.

**18.** An apparatus for processing the support of substances for detection according to claim 17, the transfer means transfers the substances for detection, together with the installing means.

**19.** An apparatus for processing support of substances for detection according to claim 18, wherein the installing section comprises a container for holding the support of substances for detection, wherein the container is communicating with a small diameter section capable of being inserted into the processing region like a vessel, and is mounted in the pipette device comprising a drawing/discharging mechanism capable of adjusting the pressure in the container, in a way that enables dismounting.

**20.** An apparatus for processing support of substances for detection according to claim 17, wherein the installing section installs the support of substances for detection according to any one of claim 5 to claim 16 in a way that the base members supported to the running support section can run, and the transfer means transfers substances for detection between the processing regions, by running only the base members in the direction of the length, in a state that the running support section is installed in the installing section.

**21.** An apparatus for processing support of substances for detection according to claim 20, wherein the transfer means comprises a running mechanism for driving to run the base member with being connected to the running support section of the support of substances for detection.

**22.** An apparatus for processing support of substances for detection according to claim 17, wherein the installing section installs the supporting member of the support of substances for detection according to any one of claim 5 to claim 16, and the transfer means comprises an inter-regions transfer means for transferring the supporting member between the processing regions together with the installing section, and a running mechanism for driving to run only the base members in the direction of the length with installing the supporting member to the installing section and connecting to the running support section, wherein the substances for detection are transferred between processing regions, by using the inter-regions transfer means and the running mechanism.

**23.** An apparatus for processing support of substances for detection according to any one of claim 17 to claim 22, wherein vessels accommodating a variety of liquid. are provided in the processing regions.

**24.** An apparatus for processing support of substances for detection according to claim 23, wherein a thermostatic control means for controlling temperature in one or more vessels is installed in one or more vessels.

**25.** An apparatus for processing support of substances for detection according to claim 24, further comprises a vibrating means for vibrating the vessel or the support of substances for detection in the vessels.

**26.** An apparatus for processing support of substances for detection according to any one of claim 23 to claim 25, wherein one or more vessels accommodate a cleaning liquid.

**27.** An apparatus for processing support of substances for detection according to the any one of claim 23 to claim 26, comprises a dry means for drying the support of substances for detection, in one of the processing regions.

**28.** An apparatus for processing support of substances for detection according to any one of claim 17 to claim 27, further comprises a detection means for detecting a change in support of substances for detection.

**29.** An apparatus for processing support of substances for detection according to the any one of claim 17 to claim 28, further comprises an analyzer for automatically, designating relevant substances for detection on the basis of locations on the base member where a change like light emission generated by a reaction in a suspension incorporating target substances, is detected, analyzing or examining a structure of the target substances etc. on the basis of a structure or characteristics of the substances for detection.

**30.** A method of processing support of substances for detection, comprising steps of: installing a support of substances for detection to an installing section in a way that enables dismounting, transferring the substances for detection between plural processing regions for process of reaction or detection, and processing the support of substances for detection in each processing region, wherein a series of process of the substances for detection can be carried out by repeating the transferring step and the processing step.

**31.** A method of processing support of substances for detection according to claim 30, wherein the substances for detection are transferred together with the installing section, at the transferring step.

**32.** A method of processing support of substances for detection according to claim 30, wherein the support of substances for detection is transferred together with an installing section, in a state that the support is installed into the installing section, at the transferring step, wherein the installing section comprises a container for holding the support of substances for detection, wherein the container is communicating with a small diameter section capable of being inserted into the processing region like a vessel, and is mounted in the pipette device comprising a drawing/discharging mechanism capable of adjusting the pressure in the container, in a way that enables dismounting.

**33.** A method of processing support of substances for detection according to claim 30, wherein the support according to any one of claim 1 to claim 16 is installed so that the base member supported to a running support section of the support of substances for detection can run at the installing step, and the substances for detection are transferred between processing regions by running only the base members in the direction of the length thereof with the support of substances for detection being installed to the installing section, at the transferring step.

**34.** A method of processing support of substances for detection according to claim 30, wherein the base members of the support of substances for detection according to any one of claim 5 to claim 16, is run in a direction of the length of the base members, and is transferred between the processing regions, by driving to run with combining a running support section to a running mechanism.

**35.** A method of processing support of substances for detection according to claim 30, wherein the supporting member of the support of substances for detection according to any one of claim 5 to claim 16, is transferred together with the installing section between the processing regions, is rotated by connecting with the running mechanism and the only base member is run and transferred in a direction of the length, so that the substances for detection are transferred between the processing regions, at the transferring step.

**36.** A method of processing support of substances for detection according to any one of claim 30 to claim 35, the processes in the processing regions are carried out in each vessel accommodating a variety of liquid.

**37.** A method of processing support of substances for detection according to any one of claim 30 to claim 36, wherein the processing step comprises a temperature control step for controlling temperature in vessels.

**38.** A method of processing support of substances for detection according to any one of claim 30 to claim 37, wherein the processing step comprises a vibrating step for vibrating the vessel or the support of substances for detection.

**39.** A method of processing support of substances for detection according to any one of claim 30 to claim 38, wherein the processing step comprises a drying step for drying the support of substances for detection to improve accuracy of detecting light emission.

**40.** A method of processing support of substances for detection according to any one of claim 30 to claim 39, wherein the processing step comprises a reacting step for reacting a suspension incorporating target substances with the substances for detection, and a detecting step for detecting a change like light emission in the support of substances for detection, further comprises an analyzing step for designating the relevant substance for detection on the basis of the detected location of the substances for detection on the base member and analyzing a structure of a target substance on the basis of the structure of the substances for detection.

**41.** A method of processing support of substances for detection according to claim 40, wherein the detection step is carried out in a state that the base member is supported to the supporting member, in a state that the base member is expanded, or in a state that the base member is running.

**42.** An apparatus for making support of substances for detection comprises,

a pipette device having one or more conduits and drawing/discharging means for adjusting a pressure in the conduits,

a regeneration section for cleaning or exchanging the conduits,

a vessel having plural liquid containing portions accommodating suspensions incorporating a variety of substances for detection and capable of being inserted by the conduit,

a stage for arranging and stretching one or more base members according to any one of the first to sixteenth aspects to be dispensed, spotted, painted, or imprinted, in parallel,

a displacement device which enables the movement of said conduits relative to the regeneration section, the vessels, the stage and the base members, and

a control section for controlling the displacement device and the drawing/discharging means,

wherein the control section controls in a manner that positions each suspension on one or more base members, in substantially vertical direction to the length of the base member, along thin parallel lines, keeping each line from contact with neighboring lines, by repeating to draw, discharge, and displace each suspension incorporating the substances for detection, and clean or exchange the conduits.

**43.** An apparatus for making support of substances for detection comprises,

a printing device having one or more conduits, one or more reservoirs accommodating suspensions incorporating various substances for detection and communicating with the conduits, and discharging means for discharging a suspension by adjusting a pressure within the conduits or reservoirs,

a regeneration section means for cleaning or exchanging the conduits and reservoirs,

a stage for arranging and stretching one or more base members according to any one of claim 1 to claim 17 to be printed by the printing device,

a displacement device which enables the movement of said conduits relative to the regeneration section, the vessels, the stage and the base members, and

a control section for controlling the displacement device and the discharging means,

wherein the control section controls in a manner that positions each suspension, on one or more base members, in substantially vertical direction to the length of the base member, along thin parallel lines, keeping each line from contact with the other lines, by repeating to discharge and displace suspensions incorporating substances for detection, and clean or exchange.

**44.** An apparatus for making support of substances for detection comprises,

one or more liquid retention tips like needles with groove, pipe, pen nib, or linear imprinting part,

a regeneration section for cleaning or exchanging the liquid retention tips,

a vessel having plural liquid containing portions accommodating suspensions incorporating a variety of substances for detection and capable of being inserted by the liquid retention tip,

a stage for arranging and stretching one or more base members according to any one of claim 1 to claim 16 to be painted, written, stained, or imprinted by the liquid retention tips in parallel,

a displacement device which enables the movement of said liquid retention tips relative to the regeneration section, the vessels, the base member, and the stage, and a control section for controlling the displacement device,

wherein the control section controls in a manner that positions each suspension, on one or more base members, in substantially vertical direction to the length of the base member, along thin parallel lines, with keeping each line from contact with the other lines, by repeating to hold, and displace each suspension incorporating the substances for detection, and clean or exchange the liquid retention tips.

**45.** An apparatus for making support of substances for detection according to claim 42 to claim 44, wherein the control section the conduits or the liquid retention tips drawing, holding or storing a particular sort of suspension so as to position each suspension incorporating a substance for detection, along a parallel line with keeping each line from contact with the other lines, by repeating to dispense, paint, imprint, stain, write, or print each sort of suspension in a direction of the length of the base members, along lines, from a fixed location on the film where one or more base members are arranged or stretched in parallel and then to exchange or clean the conduits or liquid retention tips by the regeneration section, until whole sorts of suspension are completed, while shifting the predetermined location by minute length, in order.

**46.** A method of making support of substances for detection, comprising steps of:

arranging and stretching one or more base members in parallel on a plane, positioning each substance for detection on the base members by dispensing, painting, staining, inputting, writing or printing each suspension respectively incorporating a variety of substances for detection with many parallel thin lines, with keeping each line from contact with the other lines, supporting the base members fixed by substances to a support member, wherein the fixed location of the substance for detection corresponds with the chemical structure thereof.

**47.** A method of making support of substances for detection according to claim 46, wherein the positioning step comprises steps of: drawing or holding a suspension incorporating a particular sort of substances for detection by transferring and inserting a conduits or a liquid retention tips to a vessel accommodating the suspension, dispensing, painting, staining, imputing, or writing the suspension while transferring the conduits or the liquid retention tips from a predetermined location of the base members in a direction substantially vertical to the length of the base members, and cleaning or exchanging the tube-like passengers or liquid retention tips, so that each suspension incorporating substance for detection, is positioned in parallel lines with keeping each line from contacting the other lines by repeating the above steps.

**48.** A method of making support of substances for detection according to claim 46, wherein the positioning step comprises steps of: printing a suspension incorporating a particular sort of substances for detection by transferring a conduits communicating with a reservoir accommodating the suspension, while transferring the conduits from a predetermined location of the base members in a direction substantially vertical to the length of the base members, and cleaning or exchanging the tube-like passengers and reservoirs, so that each suspension incorporating substance for detection, is positioned in parallel lines with keeping each line from contacting with the other lines by repeating the above steps.

F I G. 1

(a)
10
14
13
15
11a
11
14
15
12
13
(b)
16
14
18
15
11a
11
14
15
17
18

F I G. 2

(a)
19
14
13
15
11a
11
14
15
12
13
(b)
20
14
13
15
21a
21
14
15
12
13

FIG. 3

22
23
26
28
27
24
25
25
27
28
26
27

F I G. 4

(a)
23
37
31
30
36
22
33
32
35
(b)
23
30
22
34
34

F I G. 5

29a
21a
29
21
11a
23
11

FIG. 6

(a)
(b)
40
41
42
43
44
45
46
47
48
35
36
50
51
52
53
54
55
56
57
48
35
36

F I G. 7

(a)
(b)
69
70
68
61b
60
62
67
61
61a
66b
63
64
73
66c
66e
65
62b
62a
62
70
69
68
60
A
61
66c
66a
63
71
72
73
36
64
35
66b
66d
66e
A
65
62
48
74
75

FIG. 8

(a)

(b)

FIG. 9

80
81
83
71
84
93
36
35
85
82

FIG. 10

90
S5
94
S4
90
93
S3
S2
92
S1
92
91

F I G. 1 1

104
100
105
106
101
90
107
102
103
S11
92
91
S12
92
S13
90
93
S14
94
S15
S16

FIG. 12

(a)
(b)
124
111(112)
125
126
110
113
114
115
111
112
116
117
118
119
120
121
122
123
35
36
S26
91
92
93
94
S21
S22
S23
S24
S25

FIG. 13

(b)
127
128
63

(a)
60
61
62
63
64
65
66a
66b
66c
66d
66e
66f
66g
127
129
130
131
132
133
134

F I G. 1 4

(a)
(b)
70
69
68
150
141b
141a
67
141
152
148a
151
148
148b
147a
147b
147
143
142b
142a
142
70
69
68
140
146a
141
71
152
72
153
148
36
144
35
146c
147
146b
145
149
143
142
48

F I G. 1 5

(a)
142b
143
143
142b
142
158
156
142a
155
159
157
158
(157)
142a
(b)
142a
142b
143
142b
143
142
158
158
159
142a
158
159
156
157
155

10, 16, 19, 20, 22, 40, 50, 60, 80, 90, 110 ··· support of substances for detection

12, 17, 24, 41, 42, 61, 62, 81, 82, 141, 142 ··· reel (in which take-up reel or supply reel is included) (supporting member)

11, 21, 23, 43, 53, 63, 83, 113, 143 ··· base member

51, 111, 112 ··· drum (supporting member)

## INTERNATIONAL SEARCH REPORT

International application No.
PCT/JP01/02057

**A. CLASSIFICATION OF SUBJECT MATTER**
Int.Cl[7] G01N33/53, G01N35/02

According to International Patent Classification (IPC) or to both national classification and IPC

**B. FIELDS SEARCHED**

Minimum documentation searched (classification system followed by classification symbols)
Int.Cl[7] G01N31/22, G01N33/53, G01N35/02

Documentation searched other than minimum documentation to the extent that such documents are included in the fields searched

| | | | |
|---|---|---|---|
| Jitsuyo Shinan Koho | 1922-1996 | Toroku Jitsuyo Shinan Koho | 1994-2001 |
| Kokai Jitsuyo Shinan Koho | 1971-2001 | Jitsuyo Shinan Toroku Koho | 1996-2001 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP, 11-64322, A (Toshiba Corporation), 05 March, 1999 (05.03.99) (Family: none) | 1-5,9-11,13, 17,18,20-23,28 -31,33-36, 40,41 |
| A | | 6-8,12, 14-16,19, 24-27,32, 37-39,42-48 |
| X | JP, 48-39091, A (Scientific Industries Incorporated), 06 August, 1973 (06.08.73) (Family: none) | 1,3-5,9-11, 13,17,18, 20-22,28-31, 33-35,40,41, |
| A | | 2,6-8,12, 14-16,19, 23-27,32, 36-39,42-48 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 08 June, 2001 (08.06.01) | 19 June, 2001 (19.06.01) |
| Name and mailing address of the ISA/ Japanese Patent Office | Authorized officer |
| Facsimile No. | Telephone No. |

Form PCT/ISA/210 (second sheet) (July 1992)

INTERNATIONAL SEARCH REPORT

International application No.
PCT/JP01/02057

C (Continuation). DOCUMENTS CONSIDERED TO BE RELEVANT

| Category* | Citation of document, with indication, where appropriate, of the relevant passages | Relevant to claim No. |
|---|---|---|
| X | JP, 4-112785, A (OMRON CORPORATION), 14 April, 1992 (14.04.92) (Family: none) | 1,3-5.9-11, 13,17,18, 20-22,28,30,31 ,33-35 |
| A | | 2,6-8,12, 14-16,19, 23-27,29,32,36 -48 |
| A | JP, 58- 31998, A (Bethesda Research Laboratories Inc.), 24 February, 1983 (24.02.83) & GB, 2095833, A & DE, 3211311, A & FR, 2502785, A & US, 4446237, A | 1-48 |
| A | JP, 6-153996, A (Hitachi, Ltd.), 03 June, 1994 (03.06.94) (Family: none) | 1-48 |

Form PCT/ISA/210 (continuation of second sheet) (July 1992)